(19) Europäisches Patentamt
European Patent Office
Office européen des brevets

(11) Veröffentlichungsnummer : **0 185 130**
**B1**

(12) # EUROPÄISCHE PATENTSCHRIFT

(45) Veröffentlichungstag der Patentschrift : **02.03.88**

(21) Anmeldenummer : **85103121.1**

(22) Anmeldetag : **18.03.85**

(51) Int. Cl.⁴ : **C 07 C103/78**, A 61 K 49/04

(54) **Neue Derivate der 5-Alkoxy-2,4,6-trijod- und tribrom-isophthalsäure, Verfahren zu ihrer Herstellung und diese enthaltende Röntgenkontrastmittel.**

(30) Priorität : **10.12.84 IT 2396584**

(43) Veröffentlichungstag der Anmeldung : **25.06.86 Patentblatt 86/26**

(45) Bekanntmachung des Hinweises auf die Patenterteilung : **02.03.88 Patentblatt 88/09**

(84) Benannte Vertragsstaaten : **AT BE CH DE FR GB IT LI LU NL SE**

(56) Entgegenhaltungen : **US-A- 3 914 294**

(73) Patentinhaber : **BRACCO INDUSTRIA CHIMICA Società per Azioni**
**Via E. Folli, 50**
**I-20134 Milano (IT)**

(72) Erfinder : **Felder, Ernst, Prof. Dr.**
**Via Ceresio 49**
**CH-6826 Riva S. Vitale (CH)**
Erfinder : **Pitre, Davide, Prof. Dr.**
**Via Brocchi 24**
**Milano (IT)**
Erfinder : **Fumagalli, Luciano, Prof. Dr.**
**Via dei Guarneri 14**
**Milano (IT)**

(74) Vertreter : **Zutter, Hans Johann Niklaus**
**EPROVA Aktiengesellschaft im Laternenacker 5**
**CH-8200 Schaffhausen (CH)**

**EP 0 185 130 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die neuen als schattenbildende Bestandteile von Röntgenkontrastmittel geeigneten 5-Alkoxy-2,4,6-trijod- bzw. tribrom-isophthalsäure-diamide der allgemeinen Formel (I)

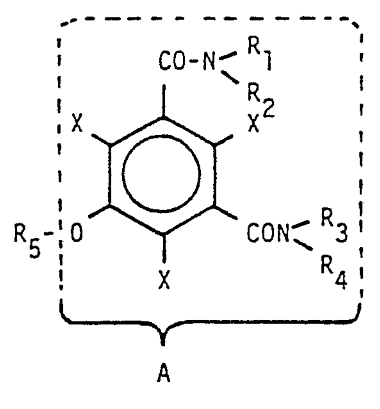

(I)

worin

X Jod oder Brom

$R_1$ und $R_3$ gleich oder verschieden sein können, nämlich Wasserstoff, Niederalkyl, Hydroxyalkyl,

$R_2$ und $R_4$ gleich oder verschieden sein können, nämlich Hydroxyalkyl mit 1 bis 5 HO-Funktionen und 2 bis 6 C-Atomen,

$R_5$ Hydroxyalkyl mit 1 bis 5 HO-Funktionen und 2 bis 6 C-Atomen, (Poly)Alkoxyalkyl mit 1 bis 10 Oxa-Funktionen und 3 bis 33 C-Atomen, Hydroxy(poly)alkoxyalkyl mit 1 bis 10 Oxa-Funktionen und 4 bis 33 C-Atomen Aminocarbonylalkyl der Formel

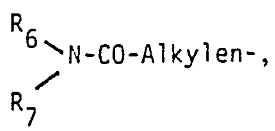

worin

$R_6$ H, Alkyl oder Hydroxyalkyl

$R_7$ Hydroxyalkyl mit 1 bis 5 HO-Funktionen und 2 bis 6 C-Atomen und

Alkylen gerades oder verzweigtes zweiwertiges Alkylen mit 1 bis 5 C-Atomen bedeutet

$R_5$ Alkyl mit 10 bzw. 20 C-Atomen oder A-Alkylen'-, worin Alkylen' zweiwertiges Alkylen mit 2 bis 12 C-Atomen mit 0 bis 5 Oxa-, Thia-, HO- oder Amid-Funktionen und A einen 3,5-Bis-(aminocarbonyl)-2,4,6-trihalogen-phenoxy-Rest der Formel I darstellt, der sich innerhalb der gestrichelten Linie befindet, bedeutet.

5-Hydroxy-2,4,6-trijod-isophthalsäure und deren Acetat sowie Ester von 5-Alkoxycarbonyl-alkoxy-2,4,6-trijod-isophthalsäure sind vor bald 20 Jahren als Röntgenkontrastmittel vorgeschlagen worden : Yoshikawa et al. Japanische Patentanmeldungen 68 02, 334-336 ; Chemical Abstracts 69 (1968) 86619t, 86643w und 86646z.

Stabilität und Verträglichkeit dieser vorbekannten Verbindungen sind ungenügend. Sie sind daher nie verwendet worden.

Die US-A-3 914 294 betrifft u. a. 5-Alkoxy-2,4,6-trijod-isophthalamsäuren, deren Salze und Ester. Diese Verbindungen sind nicht in der Lage, nicht-ionogene Röntgenkontrastmittel-Lösungen zu bilden. Die Säuren und Salze sind zwangsläufig ionogen. Ihre Ester sind nicht wasserlöslich.

Es besteht nach wie vor ein Bedürfnis an neuen gut verträglichen Röntgenkontrastmitteln, die sich in Form konzentrierter wässriger nicht-ionogener Lösungen oder als Suspensionen verwenden lassen.

Die Entwicklung in den letzten Jahren hat deutlich gezeigt, dass es beispielsweise äusserst schwierig ist und nur selten gelingt, etwa nicht-ionische Verbindungen zu finden, welche die spezifischen Eigenschaften aufweisen, die für zahlreiche Röntgenkontrastuntersuchungen höchst wünschenswert und teilweise unbedingt erforderlich sind. Solche Anforderungen sind hohe echte Wasserlöslichkeit ausreichend zur Herstellung stabiler, wenn möglich nicht übersättigter konzentrierter Lösungen, maximale allgemeine und neurotrope Verträglichkeit, ausreichend einfache Struktur für eine ökonomische Synthese sowie zur Erleichterung der Isolierung und Reinigung.

Es wurde gefunden, dass die 5-Alkoxy-2,4,6-trijod- und tribrom-isophthalsäure-diamide der Formel I den hohen Anforderungen genügen, die heute an schattengebende Komponenten in Röntgenkontrastmitteln gestellt werden.

Einige der neuen Verbindungen der Formel I gehören zur der auserlesenen Gruppe von nichtionogenen, in Wasser praktisch unbeschränkt löslichen, stabilen und gleichzeitig hochverträglichen Verbindungen, deren Dosis letalis 50 % (DL 50) nach intravenöser Applikation bei ~ 20 g/kg Maus und deren DL 50 intracerebral bei 1-2 g/kg Maus liegt.

Die Verbindungen der Formel I besitzen einen sehr breiten Anwendungsbereich. Die in Wasser sehr gut löslichen, hochverträglichen stabilen Verbindungen sind zur Herstellung von nicht-ionischen wässrigen Röntgenkontrastmitteln etwa für die Vasographie, Urographie, zur Darstellung von Körperhöhlen wie zum Beispiel Gelenkhöhlen und der Liquorräume sowie für gewisse Lymphographien geeignet.

Die in Wasser weniger gut löslichen Verbindungen dieser Reihe eignen sich zur Herstellung von Suspensionen für die Lymphographie, Hepatographie, Splenographie, Salpingographie, Bronchographie sowie die Darstellung anderer Körperhöhlen. Die Verbindungen der Formel I sind gut verträglich und werden mit der Zeit restlos aus dem Organismus ausgeschieden. Damit werden Schäden durch über Jahre liegen gebliebene Röntgenkontrastmittel, wie sie nach Verwendung etwa von Bariumsulfat und andern anorganischen Kontrastmitteln oder von jodierten Oelen beobachtet wurden, sicher vermieden.

Die Verbindungen der Formel I eignen sich auch für alle wesentlichen Anwendungsbereiche der Computer-Tomographie (Computed Tomography) und die digitale Radiographie.

Gegenstand der Erfindung sind die 5-Alkoxy-2,4,6-trijod- und tribromisophthalsäure-diamide der Formel I und Verfahren zu deren Herstellung. Gegenstand der Erfindung sind weiterhin Röntgenkontrastmittel mit einem Gehalt an mindestens einem 5-Alkoxy-2,4,6-trijod- oder tribrom-isophthalsäure-diamid der Formel I sowie deren Anwendung.

Im allgemeinen werden die Jod-Verbindungen den Brom-Verbindungen vorgezogen, da sie gewöhnlich grössere Schattendichten erzeugen.

Weiter bevorzugt werden die Diamide der allgemeinen Formel I, worin $R_1$ und $R_3$ bzw. $R_2$ und $R_4$ übereinstimmen, $R_1$ und $R_3$ gewöhnlich Wasserstoff und $R_2$ und $R_4$ niedriges Dihydroxyalkyl etwa

| | |
|---|---|
| 1,3-Dihydroxyisopropyl | $-CH(CH_2OH)_2$, |
| 2,3-Dihydroxypropyl | $-CH_2-CH(OH)-CH_2OH$, |
| 1,3-Dihydroxy-2-methyl-isopropyl | $-C(CH_2OH)_2CH_3$ |
| oder | |
| 2,3,4,5,6-Pentahydroxyhexyl | $-CH_2-(CHOH)_4-CH_2OH$ |

bedeuten.

Eine Gruppe von bevorzugten besonders stabilen, in Wasser sehr gut löslichen, hochverträglichen Verbindungen der Formel I sind die verhältnismässig einfachen 5-Hydroxyalkyl-Derivate, die zusätzliche —OH Funktionen in der Seitenkette $R_5$ aufweisen, sowie insbesondere die 5-Alkoxyalkyl- und 5-Hydroxyalkoxyalkyl-Derivate, die 2 bis 10, vorzugsweise 3 bis 8, Oxa-Funktionen in der Seitenkette enthalten. Beispiele für solche Seitenketten sind :

| | |
|---|---|
| 2,3-Dihydroxypropyl | $-CH_2-CH(OH)-CH_2OH$ |
| 1,3-Dihydroxyisopropyl | $-CH(CH_2OH)_2$ |
| 2,3,4-Trihydroxybutyl | $-CH_2(CHOH)_2CH_2OH$ |
| 2-(ω-Hydroxy-mono-, -di-, -tri-, -tetra-, -penta-, -hexa-, -hepta-(ethoxy))-ethyl | $-CH_2CH_2(OCH_2CH_2)_{1-7}-OH$ |
| 3-(ω-Hydroxy-mono-, -di-, -tri-, -tetra-, -penta-, -hexa-, -hepta-(propoxy))-propyl | $-CH_2CH_2CH_2(OCH_2CH_2CH_2)_{1-7}-OH$ |
| 3-(ω-Hydroxy-poly-(propoxy)-poly-(ethoxy))-propyl | $-CH_2CH_2CH_2(OCH_2CH_2CH_2)_{0-3}-OCH_2-CH_2)_{1-4}-(OCH_2CH_2CH_2)_{1-4}-OH$ |
| 2-(ω-Methoxy-, Ethoxy-, -mono-, -di-, -tri-, -tetra-, -penta-, -hexa-, -hepta-(ethoxy))-ethyl | $-CH_2CH_2(OCH_2CH_2)_{1-7}-OCH_3-CH_2CH_2(OCH_2CH_2)_{1-7}-OC_2H_5$ |
| 3-(ω-Methoxy-, Ethoxy-, -mono-, -di-, -tri-, -tetra-, -penta-, -hexa-, -hepta-(propoxy))-propyl | $-CH_2CH_2CH_2(OCH_2CH_2CH_2)_{1-7}-OCH_3$<br>$-CH_2CH_2CH_2(OCH_2CH_2CH_2)_{1-7}-OC_2H_5$ |

Die zahlreichen Hydroxy-Funktionen im Molekül sowie die Oxa-Funktionen verleihen den Molekülen ausgesprochen hydrophile Eigenschaften wie Wasserlöslichkeit, niedrigen Verteilerkoeffizienten Octanol/Wasser und geringe Proteinbindung.

Eine weitere Gruppe im allgemeinen in Wasser sehr gut löslichen Verbindungen der Formel I sind die 5-Aminocarbonylalkyl-Derivate, mit der Seitenkette

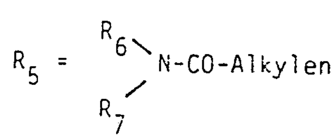

Beispiele für solche Seitenketten $R_5$ sind :

| $R_5$ | Name (der Seitenkette) |
|---|---|
| $-CH_2CONHCH_3$ | Methylaminocarbonylmethyl |
| $-CH_2CONHCH_2CH_2OH$ | 2-Hydroxyethylaminocarbonylmethyl |
| $-CH_2CONHCH_2CHOHCH_2OH$ | 2,3-Dihydroxypropylaminocarbonylmethyl |
| $-CH_2CONHCH(CH_2OH)_2$ | 1,3-Dihydroxyisopropylaminocarbonylmethyl |
| $-CHCONHCH(CH_2OH)_2$<br>$CH_3$ | 1-(1,3-Dihydroxyisopropylamino-carbonyl)ethyl |
| $CH_2OH$<br>$-CH_2CONHC-CH_3$<br>$CH_2OH$ | 1,3-Dihydroxy-2-methyl-isopropyl-aminocarbonylmethyl |
| $-CHCONHC(CH_2OH)_3$<br>$CH_3$ | 1(1,3-Dihydroxy-2-hydroxymethyl-isopropylaminocarbonyl)ethyl |
| $-CH_2CON(CH_2CH_2OH)_2$ | Bis(2-hydroxyethyl)aminocarbonylmethyl |
| $-CHCONH(CH_2CH_2OH)_2$<br>$CH_3$ | 1-(Bis-(2-hydroxyethyl)amino-carbonyl)ethyl |
| $-CH_2CONCH_2CHOHCH_2OH$<br>$CH_3$ | (N-Methyl-N-2,3-dihydroxypropyl)-aminocarbonylmethyl |
| $-CHCONCH_2CHOHCH_2OH$<br>$CH_3$ $CH_3$ | 1((N-methyl-N-2,3-dihydroxypropyl)aminocarbonyl)ethyl |
| $-CH_2CONCH_2(CHOH)_2CH_2OH$<br>$CH_3$ | (N-Methyl-N-2,3,4-trihydroxy-butyl)aminocarbonylmethyl |
| $-CHCONCH_2(CHOH)_2CH_2OH$<br>$CH_3$ $CH_3$ | 1((N-Methyl-N-2,3,4-trihydroxy-butyl)aminocarbonyl)ethyl |
| $-CH_2CONCH_2CHOHCH_2OH$<br>$CH_2CH_2OH$ | (N-2-Hydroxyethyl-N-2,3-di-hydroxypropyl)aminocarbonylmethyl |
| $-CHCON-CH_2CHOHCH_2OH$<br>$CH_3$ $CH_2CH_2OH$ | 1((N-2-Hydroxyethyl-N-2,3-di-hydroxypropyl)aminocarbonyl)ethyl |

| | |
|---|---|
| $-CH_2CONCH_2(CHOH)_3CH_2OH$<br>$CH_3$ | (N-Methyl-N-2,3,4,5-tetrahydroxypentyl)aminocarbonylmethyl |
| $-CHCONCH_2(CHOH)_3CH_2OH$<br>$CH_3$ $CH_3$ | 1((N-Methyl-N-2,3,4,5-tetrahydroxypentyl)aminocarbonyl)ethyl |
| $-CH_2CONCH_2(CHOH)_4CH_2OH$<br>$CH_3$ | (N-Methyl-N-2,3,4,5,6-pentahydroxyhexyl)aminocarbonylmethyl |
| $-CHCONCH_2(CHOH)_4CH_2OH$<br>$CH_3$ $CH_3$ | 1((N-Methyl-N-2,3,4,5,6-pentahydroxyhexyl)aminocarbonyl)ethyl |

Die Formel I umfasst auch höhere 5-Alkoxy-derivate, worin $R_5$ einen Alkyl-rest mit 10 bis 20 C-Atomen bedeutet. Diese Verbindungen enthalten einen lipophilen sowie hydrophile Reste und weisen daher grenzflächenaktive Eigenschaften auf. Sie sind besonders geeignet zur Herstellung von Suspensionen.

Weiterhin umschliesst die Formel I durch die Bedeutung von $R_5$ = A-Alkylen' auch Bis-(3,5-bis(aminocarbonyl)-2,4,6-trijod- und tribromphenoxy)-alkane vom Typus

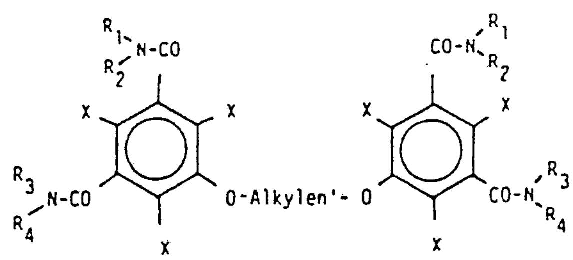

Alkylen' ist dabei zweiwertiges Alkylen, dessen Kohlenstoffkette durch Oxa-, Thia- oder Amid-Funktionen unterbrochen oder durch HO-Funktionen substituiert ist.

Beispiele für Alkylen' sind :

$-(CH_2CH_2O)_{-5}-CH_2CH_2-$, $-(CH_2CH_2CH_2O)_{0-5}-CH_2CH_2CH_2-$

$-CH_2CH_2S-CH_2CH_2-$, $-CH_2CH_2CH_2S-CH_2CH_2CH_2-$,

$-CH_2CONHCH_2CH_2NHCOCH_2-$, $-CH_2CONCH_2CH_2N-CO-CH_2-$
$CH_3$ $CH_3$

$-CH_2CONHCH_2CH-CHCH_2NHCOCH_2-$
OH OH

Obwohl Jod-Verbindungen im allgemeinen vorgezogen werden, weil sie gewöhnlich etwas dichtere Kontraste verursachen, sind auch die analogen Brom-Verbindungen sowie Mischungen von Jod- und Brom-Verbindungen von erheblichem Interesse. Sie zeichnen sich gegenüber den Jod-Verbindungen durch ihre vergleichsweise höhere Stabilität und Wasserlöslichkeit aus. Die neuen Verbindungen der Formel I sind aufgrund ihrer guten physiologischen und physikalischen Eigenschaften als schattengebende Substanzen auf praktisch allen Anwendungsgebieten von wasserlöslichen Röntgenkontrastmitteln sowie von Röntgenkontrastmittelsuspensionen gut geeignet. Sie werden gewöhnlich direkt in das zur Darstellung zu bringende Organ bzw. die entsprechende Körperhöhle appliziert oder sie werden unter Mithilfe des Organismus in das zur Darstellung zu bringende Organ transportiert.

Die Erfindung betrifft somit auch neue Röntgenkontrastmittel auf Basis von Verbindungen der allgemeinen Formel I.

Die Herstellung der neuen Röntgenkontrastmittel auf Basis der erfindungsgemässen Verbindungen der allgemeinen Formel I erfolgt in an sich bekannter Weise.

Wässrige Lösungen etwa werden bereitet indem man die schattengebende Substanz mit Wasser und den in der Galenik üblichen Zusätzen wie Stabilisatoren z. B. Di-Natrium-ethylendiamin-tetraessigsäure ($Na_2$ EDTA) oder $CaNa_2$ EDTA, physiologisch verträglichen Puffern wie z. B. teilweise versalzte Basen wie Tris(hydroxymethyl) amino-methan, Triethanolamin, 2-Amino-2-methyl-1,3-propandiol, Serinol und dergleichen in eine für die intravenöse Applikation geeignete Form bringt. Die Konzentration der Lösungen richtet sich nach den Erfordernissen der röntgendiagnostischen Methode. Die bevorzugten Konzentrationen und Dosierungen der neuen Verbindungen bewegen sich in den Bereichen von etwa 50 bis 500 mg J/ml bzw. 50 bis etwa 350 mg Br/ml für die Konzentration und 2 bis 500 ml für die Dosierung. Besonders bevorzugt sind Konzentrationen von 100 bis 400 mg J/ml bzw. 100-300 mg Br/ml.

Wässrige Suspensionen werden etwa bereitet, indem man die schattengebende Substanz mikronisiert und in Wasser, das Adjuvantien wie Kohlenhydrate, Aminosäuren, Kochsalz und Schutzkolloide wie Gelatine, Dextran, Polyvinylpyrrolidon enthält, suspendiert. Die Suspensionen enthalten in der Regel 100 bis etwa 400 mg J/ml bzw. 100 bis 300 mg Br/ml. Sie werden gewöhnlich als Lyophilisat gelagert und vor Gebrauch durch Zusatz von Wasser rekonstituiert. Weitere geeignete Darreichungsformen sind Liposome oder ultrafeine Partikel im Mikro- und Nanometer-bereich, welche die erfindungsgemässen Verbindungen als schattengebende Substanzen enthalten.

Die Herstellung der Verbindungen der allgemeinen Formel I erfolgt nach üblichen Verfahren der organischen Chemie etwa gemäss dem « allgemeinen Reaktionsschema ».

Allgemeines Reaktionsschema :

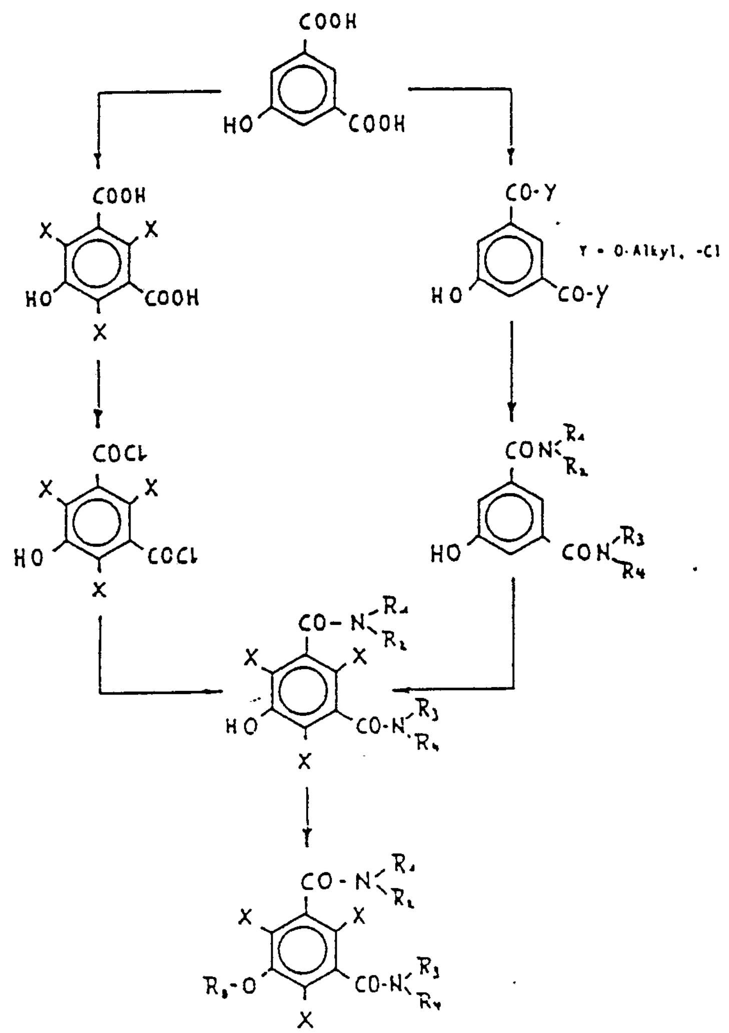


Dabei ist der letzte Syntheseschritt ein weiterer Gegenstand der vorliegenden Erfindung.

Die Erfindung betrifft demnach ferner ein Verfahren zur Herstellung von Verbindungen der allgemeinen Formel I, dadurch gekennzeichnet, dass man in an sich bekannter Weise eine Verbindung der allgemeinen Formel II

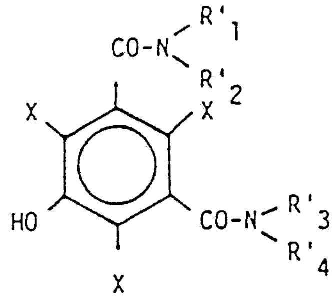

(II)

worin X, $R'_1$, $R'_2$, $R'_3$ und $R'_4$ die weiter oben genannte Bedeutung für X, $R_1$, $R_2$, $R_3$ und $R_4$ besitzt, aber im Molekül vorhandene freie Hydroxylgruppen auch in geschützter Form vorliegen können, bzw. ein Alkali- oder Erdalkalisalz davon mit einer Verbindung der allgemeinen Formel III

$$R'_5—Z \quad (III)$$

umsetzt, worin Z ein reaktives Halogenatom Cl, Br oder Jod oder ein reaktiver Sulfosäurerest bedeutet und $R'_5$ die weiter oben genannte Bedeutung für $R_5$ besitzt oder eine Alkoxycarbonylalkyl-Gruppe mit 3 bis 8 C-Atomen darstellt, und in der erhaltenen Verbindung der Formel IV

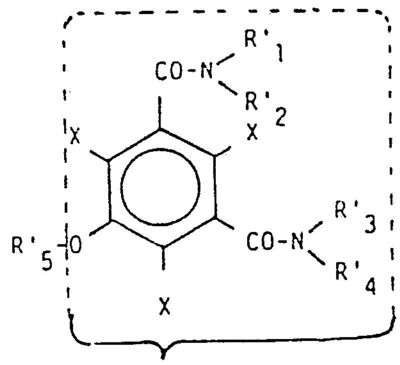

(IV)

A

vorhandene 5-Alkoxycarbonylalkoxy-gruppen durch Umsetzung mit einem primären oder sekundären Amin in die entsprechende 5-Aminocarbonylalkoxy-Gruppe überführt und schliesslich Hydroxy-Funktionen maskierende Schutzgruppen durch Hydrolyse entfernt und die Verbindung der Formel I isoliert.

Dabei ist die Umsetzung mit einem primären oder sekundären Amin nur notwendig, soweit $R'_5$ eine Alkoxycarbonylalkyl-Gruppe bedeutet. Die Hydrolyse ist nur insoweit erforderlich als maskierende Schutzgruppen vorhanden sind.

Die Veretherung durch Umsetzung von Verbindung II mit Verbindung III zu Verbindung IV erfolgt nach an sich bekannten Methoden zweckmässigerweise in einem Lösungsmittel wie Methanol, Ethanol, Isopropanol, Aceton, Methylethylketon, Methylisobutylketon, 1,2-Dimethoxyethan, Dioxan oder einem aprotischen Lösungsmittel wie Dimethylformamid (DMF), Dimethylacetamid (DMAC), Hexamethylphosphorsäuretriamid (HMPT), Acetonitril oder Dimethylsulfoxid (DMSO).

Für die Umsetzung sind insbesondere die Alkalisalze der Phenol-Derivate der Formel II geeignet, insbesondere deren Natrium-, Kalium- und Lithiumsalze.

Aber auch Erdalkalisalze (Ca, Mg, Ba) können in Betracht kommen. Eine zweckmässige Ausführungsform besteht auch darin, dass man das freie Phenol-derivat des Formel II in einem inerten Lösungsmittel wie Aceton, Methylethylketon oder DMAC mit einer Verbindung der Formel III in Gegenwart etwa von gepulvertem Kalium-, Natrium- oder Calciumcarbonat umsetzt.

Die reaktive Gruppe Z in der Verbindung der Formel III ist definitionsgemäss ein reaktives Halogenatom Chlor, Brom oder Jod oder ein reaktiver Sulfosäurerest beispielsweise Methansulfonyloxy ($MeSO_2O—$), Benzolsulfonyloxy ($PhSO_2O—$), Nitrobenzolsulfonyloxy, Toluolsulfonyloxy u. a. m. Der Rest $R'_5$ in der Verbindung der Formel III hat entweder die einangs für $R_5$ genannte Bedeutung oder stellt einen Alkoxycarbonylalkyl-rest mit 3 bis 8 C-Atomen dar. Beispiele dafür sind :

Methoxycarbonylmethyl $MeOCOCH_2—$, Ethoxycarbonylmethyl $EtOCOCH_2—$, Propoxycarbonylmethyl, 1-(Methoxycarbonyl)-ethyl $MeOCOCH(CH_3)—$, 1-(Ethoxycarbonyl)-ethyl $EtOCOCH(CH_3)—$, 2-(Ethoxycarbonyl)ethyl $EtOCOCH_2CH_2—$, 1-(Ethoxycarbonyl)-propyl $EtOCOCH(C_2H_5)_2—$, 1-(Ethoxycarbonyl)-butyl $EtOCOCH(C_3H_7)—$.

Die Veretherung von (II) zu (III) wird bei einer Temperatur reichend von Raumtemperatur bis 150 °C, vorzugsweise von 40 bis 110 °C, durchgeführt.

Die in einigen Fällen notwendige Ueberführung von Verbindung IV in Verbindung I kann umfassen :

a) Die Umsetzung eines 5-Alkoxycarbonylalkoxy-2,4,6-trihalogenisophthalsäure-diamides (der allgemeinen Formel IV) mit einem primären oder sekundären Amin in das entsprechende 5-Aminocarbonylalkoxy-2,4,6-trihalogenisophthalsäure-diamid.

b) Eine Hydrolyse zwecks Abspaltung von Hydroxy-Funktionen maskierenden Schutzgruppen.

Für die Umsetzung nach a) kommen in Betracht : Alkylamine wie z. B. Methyl- und Ethylamin oder vorzugsweise Hydroxyalkylamine wie 2-Hydroxyethylamin, Diethanolamin, 1-Amino-2,3-propandiol, 2-Amino-1,3-propandiol (Serinol), 2-Amino-2-methyl-1,3-propandiol, Tris(hydroxymethyl)-aminoethan (Tromethamin), 2-Methylamino-ethanol, 1-Methylamino-2,3-propandiol, 1-Methylamino-1-deoxy-erythrol, 1-Methylamino-1-deoxyarabit, 1-Methylamino-1-deoxy-glucitol (N-Methylglucamin), N-2-Hydroxyethyl-N-2,3-dihydroxypropylamin, N,N-Bis(2,3-dihydroxypropyl)amin, 1-Amino-1-deoxy-D-glucitol (Glucamin) u. a. m.

Diese Umsetzung wird in einem organischen Lösungsmittel wie einem niedrigen Alkohol oder einem aprotischen Lösungsmittel oder unter Umständen auch in Abwesenheit eines Lösungsmittels, z. B. in der Schmelze, bei erhöhter Temperatur von 50-150 °C, vorzugsweise 60-120 °C, durchgeführt.

Die Hydrolyse nach b) zwecks Entfernung von Schutzgruppen umfasst etwa die Spaltung von Acyloxy-gruppen zur Freisetzung der Hydroxy-gruppe durch wässrige oder wässrig-alkoholische Alkalien wie Natron- oder Kalilauge oder die Spaltung von Acetalen oder Ketalen wie insbesondere die Ueberführung von 2,2-Dimethyl-1,3-dioxan-5-ylamid-gruppen in 1,3-Dihydroxyisopropylamide oder von 2,2-Dimethyl-1,3-dioxolan-4-ylmethylamiden in 2,3-Dihydroxypropylamide durch Einwirkung von starken Säuren insbesondere von Salzsäure oder Schwefelsäure.

Die Isolierung der Verbindungen der Formel I erfolgt nach üblichen Verfahren der organischen Chemie. Bei den wasserlöslichen Verbindungen ist es oft erforderlich, vorhandene Salze, Basen oder Säuren mit Hilfe von Ionenaustauscherharzen oder durch Elektrodialyse zu entfernen.

Die folgenden Beispiele illustrieren die Erfindung.

Um die Beispiele besser lesbar zu machen, wurden für die immer wiederkehrenden Zwischenprodukte jeweils bei den Folgebeispielen Abkürzungen benutzt.

5-Hydroxy-2,4,6-trijodisophthalsäure-bis(1,3-dihydroxyisopropylamid) = HIPSA

5-Hydroxy-2,4,6-trijodisophthalsäure-bis(2,3-dihydroxypropylamid) = HIPDA

Für den Rest -2,4,6-trijodisophthalsäure-bis(1,3-dihydroxyisopropylamid) wird das Kürzel — IPSA und für den Rest -2,4,6-trijodisophthalsäure-bis(2,3-dihydroxypropylamid) das Kürzel — IPDA benutzt.

## Beispiel 1

5-(3,6-Dioxaoctyl)oxy-2,4,6-trijodisophthalsäure-bis(1,3-dihydroxyisopropylamid)

Formel I : $R_1 = R_3 = H$ ; $R_2 = R_4 = —CH(CH_2OH)_2$, $R_5 = —(CH_2CH_2O)_2C_2H_5$ ; X = J

125 g Na-Salz von 5-Hydroxy-2,4,6-trijodisophthalsäure-bis(1,3-dihydroxyisopropylamid) abgekürzt HIPSA (0,16 Mol) werden in 600 ml Dimethylacetamid (DMAC) gelöst, auf 85 °C erwärmt, mit 53 g 1(4-Nitrobenzolsulfonyloxy)-3,6-dioxaoctan versetzt und etwa 1 Stunde gerührt. Die Lösung wird im Vakuum zur Trockene verdampft, der Eindampfrückstand in 250 ml Wasser aufgenommen und durch Perkolation durch eine mit Kationenaustauscher (Amberlite(R) IR-120) sowie eine mit Anionenaustauscher (Amberlite(R) IR-45) beschickte Säule entsalzt. Das Eluat wird im Vakuum zur Trockene verdampft und wiederholt aus sehr wenig Wasser umkirstallisiert.

Die erhaltene Titelverbindung schmilzt bei 185 °C.

DC : Rf = 0,67. Laufmittel Methylethylketon/AcOH/$H_2O$ = 15 : 3 : 5

$C_{20}H_{29}J_3N_2O_9$ : J ber. 46,30 % ; gef, 46,35 %

Wasserlöslichkeit : 6 % (g/v) bei 20 °C, 100 % (g/v) bei Siedetemperatur.

Herstellung von Ausgangsmaterial und Zwischenprodukten :

A) 5-Hydroxyisophthalsäure-bis(1,3-dihydroxyisopropylamid) : 21 g 5-Hydroxyisophthalsäuredimethylester werden mit 45,5 g 2-Amino-1,3-propandiol (1,3-Dihydroxyisopropylamin) während 6 Stunden bei 90-95 °C gerührt. Der gebildete Methanol wird abdestilliert. Die Reaktionslösung wird mit Wasser verdünnt, durch Perkolation durch 300 ml Kationenaustauscher (Amberlite(R) IR 120) von überschüssigem Amin befreit und zur Trockene verdampft. Der Eindampfrückstand wird aus Methanol und/oder Ethanol umkristallisiert. Man erhält 5-Hydroxyisophthalsäure-bis(1,3-dihydroxyisopropylamid) vom Schmelzpunkt 199-200 °C.

$C_{14}H_{20}N_2O_7$ : N ber. 8,53 % ; gef. 8,68 %.

B) 5-Hydroxy-2,4,6-trijodisophthalsäure-bis(1,3-dihydroxyisopropylamid) (HIPSA)

65,6 g 5-Hydroxyisophthalsäure-bis(1,3-dihydroxyisopropylamid) (0,2 Mol) werden in 670 ml Wasser durch Zusatz von 30 %iger wässeriger Natronlauge bis zum pH von 9,4 in Lösung gebracht. Nun werden 323 g einer wässrigen 24 % Jod enthaltenden Natriumjoddichlorid ($NaJCl_2$)-Lösung (0,61 Mol) innert 4 Stunden unter Rühren eingetropft, wobei der pH der Reaktionslösung durch Zusatz von Natronlauge zwischen 9,4 und 9,7 gehalten wird.

Man rührt anschliessend noch 6 Stunden bei 20 °C und fällt das Produkt durch Zusatz von Salzsäure und entfärbt es gleichzeitig mit 2 g Natriumhydrogensulfit.

Nach Umfällen aus verdünnter wässriger Natronlauge mit Salzsäure erhält man 115,5 g, das sind 81,8 % der Theorie 5-Hydroxy-2,4,6-trijodisophthalsäure-bis(1,3-dihydroxyisopropylamid) vom Schmelzpunkt 175-180 °C. Aequivalentgewicht : ber. 706,0 ; gef. 702,5.

113 g (= 0,16 Mol) dieses Trijodphenol-Derivates werden in 250 ml Methanol suspendiert und mit 33 ml 5,35 molarem methanolischem Natriummethylat versetzt. Dabei wird zunächst eine klare Lösung erhalten, aus welcher nach einiger Zeit das Na-Salz(Na-Phenolat) von 5-Hydroxy-2,4,6-trijodisophthalsäure-bis(1,3-dihydroxyisopropylamid) (Na-HIPSA) auskristallisiert.

C) 1-(4-Nitrobenzolsulfonyloxy)-3,6-dioxaoctan : 44,2 g Diethylenglykolmonoethylether (0,32 Mol) in 300 ml Ethylacetat werden mit 32,1 g Triethylamin (0,31 Mol) und danach unter Rühren in 3 Portionen mit insgesamt 60,3 g 4-Nitrobenzolsulfonylchlorid (0,3 Mol) versetzt.

Das ausgeschiedene Triethylamin-hydrochlorid wird abfiltriert und aus dem Filtrat das Titel-Produkt isoliert.

Schmelzpunkt : 47-48 °C.

## Beispiel 2

5-(3,6,9-Trioxadecyl) oxy-2,4,6-trijodisophthalsäure-bis(1,3-dihydroxy-isopropylamid)

Formel 1 : $R_1=R_3=—H$ ; $R_2=R_4=—CH(CH_2OH)_2$ ; $R_5(CH_2CH_2O)_3—CH_3$ ; X = J

115,6 g Na-Salz von HIPSA (=NA-HISPA) werden mit 72 g 1(4-Nitrobenzolsulfonyloxy)-3,6,9-trioxadecan, ähnlich wie im Beispiel 1 beschrieben, umgesetzt und aufgearbeitet und schliesslich aus Ethanol umkristallisiert.

Schmelzpunkt : 90-91 °C

DC : Rf = 0,5. Laufmittel $CHCl_3/MeOH/NH_4OH$ (25 %ig) = 6 : 3 : 1.

$C_{21}H_{31}J_3N_2O_{10}$ : J ber. 44,67 % ; gef. 44,74 %.

Wasserlöslichkeit : ≥ 100 % (g/v) bei Raumtemperatur.

1(4-Nitrobenzolsulfonyloxy)-3,6,9-trioxadecan wird durch Umsetzung von 4-Nitrobenzolsulfonylchlorid (99,5 g) mit Triethylenglykolmonomethylether (90) in Gegenwart von Triethylamin (48 g) hergestellt. Schmelzpunkt (nach Umkristallisieren aus Ethanol) 45 °C.

## Beispiel 3

5-(3,6,9,12,15-Pentaoxahexadecyl) oxy-2,4,6-trijod-isophthalsäure-bis-(1,3-dihydroxyisopropylamid)

Formel I : $R_1=R_2=H$ ; $R_2=R_4=—CH(CH_2OH)_2$ ; $R_5=—(CH_2CH_2O)_5—CH_3$ ; X = J

47,5 g Na-HIPSA werden mit 31,4 g chromatographisch gereinigtem 1(4-Nitrobenzolsulfonyloxy)-3,6,9,12,15-pentaoxahexadecan) umgesetzt.

Schmelzpunkt : 110-112 °C.

DC : Rf = 0,45. Laufmittel : $CHCl_3/MeOH/NH_4OH$ = 6 : 3 : 1

$C_{25}H_{39}J_3N_2O_{12}$ : J ber. 40,49 % ; gef. 40,44 %

Wasserlöslichkeit : ≥ 100 % (g/v) bei Raumtemperatur.

1-(4-Nitrobenzolsulfonyloxy)-3,6,9,12,15-pentaoxahexadecan wurde auch durch die äquivalente Menge 1-Jod-3,6,9,12,15-pentaoxahexadecan ersetzt.

## Beispiel 4

5-(3,6,9,12,15,18,21-Heptaoxadocosyl) oxy-2,4,6-trijodisophthalsäure-bis(1,3-dihydroxyisopropylamid)

Formel I : $R_1=R_3=H$ ; $R_2=R_4=—CH(CH_2OH)_2$ ; $R_5=—(CH_2CH_2O)_7—CH_3$ ; X = J

29,2 g Na-HIPSA werden mit 22 g chromatographisch gereinigtem 1(4-Nitrobenzolsulfonyloxy)-3,6,9,12,15,18,21-heptaoxadocosan umgesetzt. Salze werden mittels Ionenaustauscher entfernt, das Produkt wird durch fraktionierte Fällung etwa aus Ethylacetat mit Benzin gereinigt.

DC : Rf = 0,62 ; Laufmittel : $CHCl_3/MeOH/NH_4OH$ = 6 : 3 : 1

$C_{29}H_{47}J_3N_2O_{14}$ : J ber. 37,02 % ; gef. 37,18 %.

Wasserlöslichkeit : ≥ 100 % (g/v) bei 20 °C.

(1-(4-Nitrobenzolsulfonyloxy)-3,6,9,12,15,18,21-heptaoxadocosan wurde mit Erfolg auch durch die äquivalente Menge 1-Brom-3,6,9,12,15,18,21-heptooxadocosan ersetzt.

## Beispiel 5

5-(8-Hydroxy-3,6-dioxaoctyl) oxy-2,4,6-trijodisophthalsäure-bis(1,3-dihydroxyisopropylamid

Formel I : $R_1=R_3=H$ ; $R_2=R_4=-CH(CH_2OH)_2$ ; $R_5=-CH_2CH_2(OCH_2CH_2)_2-OH$ ; X = J

205 g Na-HIPSA werden in 600 ml DMAC mit 93,8 g 1(4-Nitrobenzolsulfonyloxy)-3,6-dioxaoctan-8-ol, ähnlich wie im Beispiel 1 beschrieben, umgesetzt und aufgearbeitet.

Schmelzpunkt (nach Umkristallisation aus Ethanol) : 120 °C.

DC : Rf = 0,38. Laufmittel : $CHCl_3/MeOH/NH_4OH$ = 6 : 3 : 1.

$C_{20}H_{29}J_3N_2O_{10}$ : ber. 45,42 % ; gef. 45,40 %.

Wasserlöslichkeit : ≥ 100 % (g/v) bei Raumtemperatur.

1-(4-Nitrobenzolsulfonyloxy)-3,6-dioxaoctan-8-ol : 204 g Triethylenglykol (1,35 Mol) werden in eine benzolische Lösung enthaltend 66,3 g 4-Nitrobenzolsulfonylchlorid (0,3 Mol) und 47,9 g Pyridin (0,6 Mol) unter Rühren eingetropft. Das erhaltene Produkt kann durch einfache Chromatographie an Silicagel gereinigt werden.

DC : Rf = 0,4. Laufmittel : Butylacetat/AcOH/$H_2O$ = 5 : 1 : 1 (obere Phase).

## Beispiel 6

5-(16-Hydroxy-4,7,10,13-tetraoxahexadecyl) oxy-2,4,6-trijod-isophthalsäure-bis(1,3-dihydroxyisopropylamid)

Formel I : $R_1=R_3=-H$ ; $R_2=R_4=-CH(CH_2OH)$ ;
$R_5=-CH_2CH_2CH_2O(CH_2CH_2O)_3-CH_2CH_2CH_2OH$ ; X = J

36 g Na-HIPSA werden in einem aprotischen Lösungsmittel (DMAC, DMF, HMPT, DMSO oder Acetonitril mit 26,5 g 1-(4-Nitrobenzolsulfonyloxy)-4,7,10,13-tetraoxadecan-16-ol bei 40-110 °C umgesetzt. Das Produkt lässt sich aus Ethanol umkristallisieren.

Schmelzpunkt : 96-100 °C

DC : Rf = 0,49. Laufmittel $CHCl_3/MeOH/MH_4OH$ = 6 : 3 : 1.

$C_{26}H_{41}J_3N_2O_{12}$ : J ber. 39,89 % ; gef. 39,80 %.

Wasserlöslichkeit : ≥ 100 % (g/v) bei 20 °C.

## Beispiel 7

5-(3,6,9-Trioxadecyl) oxy-2,4,6-tribromisophthalsäure-bis(1,3-dihydroxyisopropylamid)

Formel I : $R_1=R_3=-H$ ; $R_2=R_4=-CH(CH_2OH)_2$ ; $R_5=-(CH_2CH_2O)_3CH_3$ ; X = Br

20 g Na-Salz von 5-Hydroxy-2,4,6-tribrom-isophthalsäure-bis(1,3-dihydroxyisopropylamid) werden in einem aprotischen Lösungsmittel mit 11,9 g 1-(4-Nitrobenzolsulfonyloxy)-3,6,9-trioxadecan oder mit 7,7 g 1-Brom-3,6,9-trioxadecan umgesetzt.

Schmelzpunkt : 57-59 °C

DC : Rf = 0,5. Laufmittel $CHCl_3/MeOH/NH_4OH$ = 6 : 3 : 1.

$C_{21}H_{31}Br_3N_2O_{10}$ : Br ber. 33,70 % ; gef. 33,15 %.

Wasserlöslichkeit : ≥ 100 % (g/v) bei 20 °C.

Herstellung von Ausgangsmaterial und Zwischenprodukten :

A) 5-Hydroxy-2,4,6-tribromisophthalsäure :

109,2 g 5-Hydroxyisophthalsäure werden in Wasser unter Rühren mit 300 g Brom behandelt. Die erhaltene 5-Hydroxy-2,4,6-tribromisophthalsäure schmilzt bei 280 °C.

B) 5-Hydroxy-2,4,6-tribromisophthalsäure-bis(1,3-dihydroxyisopropylamid) :

5-Hydroxy-2,4,6-tribromisophthalsäure wird durch Behandlung mit einem Ueberschuss von Thionylchlorid in ihr Säurechlorid übergeführt.

205 g 5-Hydroxy-2,4,6-tribromisophthalsäuredichlorid werden in DMAC mit überschüssigem 2-Amino-1,3-propandiol (Serinol) umgesetzt. Gebildetes Serinol-Hydrochlorid und überschüssiges Amin werden mittels Ionenaustauscher entfernt. Das Rohprodukt wird aus wenig Wasser umkristallisiert.

5-Hydroxy-2,4,6-tribromisophthalsäure-bis(1,3-dihydroxyisopropylamid) schmilzt bei 225 °C.

## Beispiel 8

5-(2,3-Dihydroxypropyl) oxy-2,4,6-trijodisophthalsäure-bis(1,3-dihydroxyisopropylamid)

Formel I : $R_1=R_3=-H$ ; $R_3=R_4=-CH(CH_2OH)_2$ ; $R_5=-CH_2CH(OH)\ CH_2OH$ ; X = J

125 g Na-HIPSA werden in siedendem Ethanol mit 22 g 3-Chlor-1,2-propandiol umgesetzt und, ähnlich wie im Beispiel 1 beschrieben, aufgearbeitet.

Erhaltenes Titelprodukt ist löslich in Wasser.

Beispiel 9

5-(1,3-Dihydroxyisopropyl) oxy-2,4,6-trijodisophthalsäure-bis(1,3-dihydroxyisopropylamid)

Formel I : $R_1=R_3=$—H ; $R_3=R_4=R_5=$—$CH(CH_2OH)_2$ ; X = J

12,5 g Na-HIPSA werden in siedendem Ethanol mit 5 g 1-(4-Toluolsulfonyloxy)-1,3-propandiol umgesetzt.

Das erhaltene Titelprodukt ist in Wasser leicht löslich.

Beispiel 10

5-(2,2-Bis-(hydroxymethyl)-3-hydroxypropyl) oxy-2,4,6-trijodisophthalsäure-bis(1,3-dihydroxyisopropylamid)

Formel I : $R_1=R_3=$—H ; $R_2=R_4=$—$CH(CH_2OH)$ ; $R_5=$—$CH_2$—$C(CH_2OH)_3$ ; X = J

12,5 g Na-HIPSA werden mit 3 g 2-Chlormethyl-2-hydroxymethylpropan-1,3-diol umgesetzt.

Erhaltenes Titelprodukt ist spielend leicht wasserlöslich.

Beispiel 11

5-Methylaminocarbonylmethoxy-2,4,6-trijod-isophthalsäure-bis(1,3-dihydroxyisopropylamid)

Formel I : $R_1=R_3=$—H ; $R_2=R_4=$—$CH(CH_2OH)_2$ ; $R_5=$—$CH_2CONHMe$ ; X = J

95 g 5-Methoxycarbonylmethoxy-2,4,6-trijodisophthalsäure-bis(1,3-dihydroxyisopropylamid) (= 5-Methoxycarbonylmethoxy-IPSA) werden in Methanol bei 40-70 °C mit überschüssigem wässrigem Methylamin behandelt. Nach dem Eindampfen zur Trockene und Umkristallisieren aus Wasser erhält man reine Titelverbindung.

Schmelzpunkt : 280 °C unter Zersetzung.

DC : Rf = 0,34. Laufmittel : $CHCl_3/MeOH/NH_4OH$ = 6 : 3 : 1

$C_{17}H_{22}J_3N_3O_8$ : J ber. 48,99 % ; gef. 48,91 %

Wasserlöslichkeit : 10 % (g/v) bei Siedetemperatur.

5-Methoxycarbonylmethoxy-2,4,6-trijodisophthalsäure-bis(1,3-dihydroxyisopropylamid) (= 5-Methoxycarbonylmethoxy-IPSA) wird erhalten durch Umsetzung von Na-HIPSA (240 g) mit 75 g Bromessigsäuremethylester in DMAC. Schmelzpunkt nach Umkristallisation aus Wasser : 155 °C.

Beispiel 12

5-(1,3-Dihydroxyisopropylaminocarbonyl) methoxy-2,4,6-tribromisophthalsäure-bis(1,3-dihydroxyisopropylamid)

Formel I : $R_1=R_3=$—H ; $R_2=R_4=$—$CH(CH_2OH)_2$ ; $R_5=$—$CH_2CONHCH(CHOH)_2$ ; X = Br

80 g Na-Salz von 5-Hydroxy-2,4,6-tribromisophthalsäure-bis(1,3-di-hydroxyisopropylamid) in 450 ml DMAC werden bei 80 °C mit 24,5 g Bromessigsäuremethylester umgesetzt. Erhaltenes rohes 5-Methoxycarbonylmethoxy-2,4,6-tribromisophthalsäure-bis(1,3-dihydroxyisopropylamid) wird mit 24,5 g Serinol (2-Amino-1,3-propandiol) bei 80 °C umgesetzt. Nach dem Eindampfen zur Trockene werden gebildete Salze und überschüssiges Serinol mit Hilfe eines Kationen- und eines Anionenaustauschers entfernt. Erhaltenes Rohprodukt wird aus Methanol umkristallisiert.

Schmelzpunkt : 220 °C.

DC : Rf = 0,48. Laufmittel : Methylethylketon/AcOTEt/$H_2O$ = 15 : 3 : 5

$C_{19}H_{26}Br_3N_3O_{10}$ : Br ber. 34,43 % ; gef. 34,20 %.

Wasserlöslichkeit : $\geqslant$ 100 % (g/v) bei 20 °C.

Das als Ausgangsmaterial benutzte Na-Salz wird aus dem entsprechenden Tribromphenol-Derivat durch Behandlung mit der äquivalenten Menge Natriummethylat in wenig Methanol erhalten.

Beispiel 13

R(+)-5-(1(1,3-Dihydroxyisopropylaminocarbonyl) ethoxy)-2,4,6-tribromisophthalsäure-bis(1,3-dihydroxyisopropylamid)

$$\text{Formel I: } R_1=R_3=H;\ R_2=R_4= -CH(CH_2OH)_2;\ R_5= -\overset{\displaystyle CH_3}{\overset{|}{\underset{*}{C}}}HCONHCH(CH_2OH)_2;\ X = Br$$

25 g R(+)-5-(1(Methoxycarbonyl) ethoxy)-2,4,6-tribrom-isophthalsäure-bis(1,3-dihydroxyisopropylamid) werden in 100 ml Methanol mit 7 g Serinol versetzt. Die Reaktionslösung wird während 18 Stunden am Rückfluss gekocht. Das Produkt kristallisiert aus. Es lässt sich aus Wasser oder viel Methanol umkristallisieren.

Schmelzpunkt : 225-230 °C

DC : Rf = 0,46. Laufmittel 2-Butanon/AcOH/$H_2O$ = 15 : 3 : 5

$C_{20}H_{28}Br_3N_3O_{10}$ : Br ber. 33,45 % ; gef. 33,50 %

$[\alpha]_{436}^{20}$=—22,19° (c = 5 % in DMSO)

Wasserlöslichkeit : 5 % (g/v) bei 20 °C, 100 % (g/v) bei 100 °C.

R(+)-5(1(Methoxycarbonyl) ethoxy)-2,4,6-tribromisophthalsäure-bis(1,3-dihydroxyisopropylamid) :

58,7 g Na-Salz von 5-Hydroxy-2,4,6-tribromisophthalsäure-bis(1,3-dihydroxyisopropylamid) (0,1 Mol) werden mit 28,9 g S(—)-2(4-Nitrobenzolsulfonyloxy) propionsäuremethylester (hergestellt durch Umsetzung von (S)-Milchsäuremethylester mit 4-Nitrobenzolsulfonylchlorid) in DMAC bei 70 °C umgesetzt.

Die sehr gut wasserlösliche Titelverbindung schmilzt bei ~ 160-170 °C ;

$[\alpha]_{436}^{20}$ = + 24,89 % (c = 5 % in Wasser).

Beispiel 14

R(—)-5-(1(1,3-Dihydroxyisopropylaminocarbonyl) ethoxy)-2,4,6-trijodisophthalsäure-bis(1,3-dihydroxyisopropylamid)

$$\text{Formel I: } R_1=R_3= H;\ R_2=R_4= -CH(CH_2OH)_2;\ R_5= -\overset{\displaystyle CH_3}{\overset{|}{\underset{*}{C}}}HCONHCH(CH_2OH)_2;\ X = J$$

74 g R(+)-5(1(Methoxycarbonyl) ethoxy-IPSA werden mit 17 g Serinol in siedendem Methanol umgesetzt.

Die erhaltene Titelverbindung schmilzt bei 250 °C.

$C_{20}H_{28}J_3N_3O_{10}$ : J ber. 44,73 % ; gef. 44,85 %

DC : Rf = 0,35. Laufmittel AcOEt/AcOH/$H_2O$ = 20 : 10 : 6

$[\alpha]_{436}^{20}$ = — 48,5° (c = 5 % in DMSO)

Wasserlöslichkeit : gering.

R(+)-5(1(Methoxycarbonyl) ethoxy)-IPSA :

120 g Na-HIPSA und 47,7 g S(—)-2(4-Nitrobenzolsulfonyloxy)-propionsäuremetylester werden in DMF bei 40-80 °C umgesetzt.

Schmelzpunkt 95-100 °C, $[\alpha]_{436}^{20}$ = + 6,12 (c = 5 % in Wasser)

Wasserlöslichkeit : ~ 80 % (g/v) bei 25 °C.

Beispiel 15

5-(1,3-Dihydroxy-2-methylisopropylaminocarbonyl) methoxy-2,4,6-trijodisophthalsäure-bis(1,3-dihydroxyisopropylamid)

Formel I : $R_1=R_3=H$ ; $R_2=H_4$=—$CH(CH_2OH)_2$ ; $R_5$=—$CH_2CONHC(CH_3)$ $(CH_2OH)_2$, X = J

11,67 g 5-Methoxycarbonylmethoxy-IPSA (0,15 Mol) werden mit 47,2 g 1,1-Bis(hydroxymethyl) ethylamin (0,45 Mol) in 600 ml Methanol während 4 Stunden bei 85 °C gerührt. Das Lösungsmittel wird verdampft, der Rückstand in Wasser gelöst und mit Kationen- und Anionenaustauscherharz behandelt. Das Eluat wird zur Trockene verdampft und aus Wasser umkristallisiert.

Die erhaltene Titelverbindung schmilzt bei : 225-230 °C

DC : Rf = 0,63. Laufmittel $CHCl_3/MeOH/NH_4OH$=2 : 2 : 1

$C_{20}H_{28}J_3N_3O_{10}$ : J ber. 44,72 % ; gef. 44,55 %.

Wasserlöslichkeit : 1,6 % (g/v) bei 20 °C, 100 % (g/v) bei Siedetemperatur.

Beispiel 16

R(—)-5(1(1,3-Dihydroxy-2-hydroxymethyl-isopropylaminocarbonyl) ethoxy)-2,4,6-trijodisophthalsäure-bis(1,3-dihydroxyisopropylamid)

$$\text{Formel I: } R_1=R_3= H;\ R_2=R_4= -CH(CH_2OH)_2;\ R_5= -\underset{\displaystyle CH_3}{\underset{|}{\overset{*}{C}}}HCONHC(CH_2OH)_3;\ X = J$$

85 g R(+)-5(1-Methoxycarbonyl) ethoxy-IPSA (0,107 Mol) werden in Methanol mit 25,8 g Tris(hydroxymethyl) aminomethan (2-Amino-2-hydroxymethyl-1,3-propandiol), ähnlich wie im Beispiel 14 beschrieben, umgesetzt und aufgearbeitet.

Die erhaltene Titelverbindung schmilzt bei 220-230 °C unter Zersetzung.
DC : Rf = 0,38. Laufmittel AcOEt/AcOH/$H_2O$=20 : 10 : 6
$C_{21}H_{30}J_3N_3O_{11}$ : J ber. 43,19 % ; gef. 42,70 %.
$[\alpha]_{436}^{20}$ = — 61.7° (c = 5 % in Wasser)
Wasserlöslichkeit : 2,5 % (g/v) bei 25 °C, 100 % (g/v) bei 100 °C.

## Beispiel 17

5-(Bis(2-hydroxyethyl) aminocarbonylmethoxy-2,4,6-trijodisophthalsäure-bis(1,3-dihydroxyisopropylamid)

Formel 1 : $R_1$=$R_3$=H ; $R_2$=$R_4$=—$CH(CH_2OH)_2$ ; $R_5$=—$CH_2CON(CH_2CH_2OH)_2$ ; X=J

155,6 g 5-Methoxycarbonylmethoxy-IPSA (0,2 Mol) werden mit 84 g Diethanolamin (0,8 Mol) in Methanol umgesetzt. Die erhaltene Titelverbindung schmilzt nach Umkristallisieren aus trockenem Ethanol bei 170-180 °C.
DC : Rf = 0,5. Laufmittel $CHCl_3$(MeOH/$NH_4OH$=2 : 2 : 1
$C_{20}H_{28}J_3N_3O_{10}$ : J ber. 44,72 % ; gef. 44,65 %.
Wasserlöslichkeit : ≥ 100 % (g/v) bei 25 °C.

## Beispiel 18

R,S-5-(1-Bis-2-hydroxyethyl) aminocarbonyl) ethoxy)-2,4,6-trijodisophthalsäure-bis(1,3-dihydroxyisopropylamid)

$$\text{Formel I: } R_1=R_3=\text{H};\ R_2=R_4=\text{-CH(CH}_2\text{OH)}_2;\ R_5=\text{-}\underset{}{\overset{CH_3}{\overset{|}{C}}}\text{H-CON(CH}_2\text{CH}_2\text{OH)}_2;\ X = J$$

85 g R,S-5-(1(Methoxycarbonyl) ethoxy)-IPSA (0,107 Mol) werden in Ethanol mit 45 g Diethanolamin (0,43 Mol) umgesetzt.
Die erhaltene Titelverbindung schmilzt bei ca 170-180 °C.
DC : Rf = 0,61. Laufmittel $CHCl_3$/MeOH/$NH_4OH$=2 : 2 : 1
$C_{21}H_{30}J_3N_3O_{10}$ : J ber. 44,00 % ; gef. 43,65 %.
Wasserlöslichkeit : ≥ 100 % (g/v) bei 25 °C.

## Beispiel 19

5-(N-Methyl-N-2,3-dihydroxypropyl) aminocarbonylmethoxy-2,4,6-trijodisophthalsäure-bis-(1,3-dihydroxyisopropylamid

$$\text{Formel I: } R_1=R_3=\text{H};\ R_2=R_4=\text{-CH(CH}_2\text{OH)}_2;\ R_5=\text{-CH}_2\text{CO}\overset{CH_3}{\overset{|}{N}}\text{CH}_2\text{CH(OH)CH}_2\text{OH};\ X = J$$

376 g 5-Methoxycarbonylmethoxy-IPSA (0,483 Mol) werden mit 135 g 1-Methylamino-2,3-propandiol bei 80-100 °C umgesetzt.
Die erhaltene Titelverbindung schmilzt bei ca 200 °C.
DC : Rf = 0,67. Laufmittel $CHCl_3$/MeOH/$NH_4OH$=2 : 2 : 1
$C_{20}H_{28}J_3N_3O_{10}$ : J ber. 44,72 %, gef. 44,30 %.
Wasserlöslichkeit : ≥ 100 % (g/v) bei 20 °C.

## Beispiel 20

R,S und R-5-(1(N-Methyl-N-2,3-dihydroxypropylamino) carbonyl) ethoxy-2,4,6-trijodisophthalsäure-bis(1,3-dihydroxyisopropylamid)

Formel I: $R_1=R_3=$ H; $R_2=R_4=$ $-CH(CH_2OH)_2$; $R_5=$ $-\underset{CH_3}{CH}CO\underset{CH_3}{N}CH_2CH(OH)CH_2OH$; X = J

Je 95 g R,S und R-5(1(Ethoxycarbonyl) ethoxy)-IPSA (0,118 Mol) werden in trockenem Ethanol jeweils mit 32,5 g 1-Methylamino-2,3-propandiol bei 100 °C umgesetzt.
Die erhaltene R,S-Titelverbindung schmilzt bei etwa 150 °C.
DC : Rf = 0,22. Laufmittel AcOEt/AcOH/$H_2O$=20 : 10 : 6
$C_{21}H_{30}J_3N_3O_{10}$ : J ber. 44,00 ; gef. 43,96 %.
Wasserlöslichkeit : ≥ 100 % (g/v) bei 20 °C.
Die erhaltene optische aktive R-Form schmilzt bei etwa 130 °C.
DC : Rf = 0,33. Laufmittel AcOEt/AcOH/$H_2O$=20 : 10 : 6.
J : gef. 42,99 % ; $[\alpha]_{436}^{20}$ = + 21,9° (c = 2 % in MeOH)
Wasserlöslichkeit : ≥ 100 % (g/v) bei 20 °C.

## Beispiel 21

5-(N-Methyl-N-2,3,4-trihydroxybutyl) aminocarbonylmethoxy-2,4,6-trijodisophthalsäure-bis(1,3-dihydroxyisopropylamid)

Formel I: $R_1=R_3=$ H; $R_2=R_4=$ $-CH(CH_2OH)_2$; R= $-CH_2CO\underset{CH_3}{N}CH_2(CHOH)_2CH_2OH$;
X = J

140 g 5-Methoxycarbonylmethoxy-IPSA (0,18 Mol) und 48,5 g 1-Methylamino-1-deoxy-D-erythrol (0,36 Mol) werden in Methanol oder Ethanol einige Stunden am Rückflusskühler gekocht.
Die erhaltene funktionell gereinigte, amorphe Titelverbindung schmilzt bei ca 150 °C.
DC : Rf = 0,27. Laufmittel Methylethylketon/AcOH/$H_2O$=15 : 3 : 5
$C_{21}H_{30}J_3N_3O_{11}$ : J ber. 43,20 % ; gef. 42,95 %.
$[\alpha]_{436}^{20}$ = — 4,8° (c = 2 in MeOH)
Wasserlöslichkeit : ≥ 100 % (g/v) bei 20 °C.

## Beispiel 22

(5-(1(N-Methyl-N-2,3,4-trihydroxybutyl) aminocarbonyl) ethoxy)-2,4,6-trijodisophthalsäure-bis(1,3-dihydroxyisopropylamid)

Formel I: $R_1=R_3=$ H; $R_2=R_4=$ $-CH(CH_2OH)_2$; $R_5=$ $-\underset{CH_3}{\overset{*}{C}H}CO\underset{CH_3}{N}CH_2(CHOH)_2CH_2OH$; X = J

230 g R-5(1(Ethoxycarbonyl) ethoxy-IPSA (0,285 Mol) und 99,5 g 1-Methylamino-1-deoxy-D-erythrol (0,736 Mol) werden in Ethanol während 100 Stunden am Rückfluss gekocht.
Die funktionell gereinigte Titelverbindung schmilzt bei etwa 170 °C.
DC : Rf = 0,39. Laufmittel 2-Butanon/AcOH/$H_2O$=15 : 3 : 5
$C_{22}H_{32}J_3N_3O_{11}$ : J ber. 42,52 % ; gef. 42,50 %.
$[\alpha]_{436}^{20}$ = — 2,8° (c = 2 % in MeOH)
Wasserlöslichkeit : ≥ 100 % (g/v) bei 25 °C.

## Beispiel 23

5-(N-2-Hydroxyethyl-N-2,3-dihydroxypropyl) aminocarbonylmethoxy-2,4,6-trijodisophthalsäure-bis(1,3-dihydroxyisopropylamid)

Formel I: $R_1=R_3=$ H; $R_2=R_4=$ $-CH(CH_2OH)_2$; $R_5=$ $-CH_2CO\underset{CH_2CH_2OH}{N}CH_2CHOHCH_2OH$; X = J

155,6 g 5-Methoxycarbonylmethoxy-IPSA (0,2 Mol) werden mit 54 g N-2-Hydroxyethyl-N-2,3-dihydroxypropylamin (0,4 Mol) umgesetzt.
Die erhaltene Titelverbindung ist spielend leicht löslich in Wasser (≥ 100 % (g/v)).
DC : Rf = 0,49. Laufmittel $CHCl_3$/MeOH/$NH_4OH$=2 : 2 : 1

## Beispiel 24

R-5(1((N-2-Hydroxyethyl-N-2,3-dihydroxypropyl) aminocarbonyl) ethoxy)-2,4,6-trijodisophthalsäure-bis(1,3-dihydroxyisopropylamid)

Formel I: $R_1=R_3$; $R_2=R_4= -CH(CH_2OH)_2$; $R_5= -\overset{*}{C}HCONCH_2CHOHCH_2OH$; X = J
$\qquad\qquad\qquad\qquad\qquad\qquad\qquad\qquad\quad CH_3\ \ CH_2CH_2OH$

205 g R-5(1(Ethoxycarbonyl) ethoxy)-IPSA (0,25 Mol) werden mit 67,5 g N-2-Hydroxyethyl-N-2,3-dihydroxypropylamin (0,5 Mol) umgesetzt.

Die erhaltene Titelverbindung ist spielend leicht löslich in Wasser (⩾ 100 % (g/v)).

DC : Rf = 0,40. Laufmittel Methylethylketon/AcOH/$H_2O$=15 : 3 : 5

$[\alpha]_{436}^{20} = + 56{,}6$ (c = 2 % in MeOH).

## Beispiel 25

5-(N-Methyl-N-2,3,4,5-tetrahydroxypentyl) aminocarbonylmethoxy-2,4,6-trijodisophthalsäure-bis(1,3-dihydroxyisopropylamid)

Formel I: $R_1=R_3=$ H; $R_2=R_4= -CH(CH_2OH)_2$; $R_5= -CH_2CONCH_2(CHOH)_3CH_2OH$;
X = J $\qquad\qquad\qquad\qquad\qquad\qquad\qquad CH_3$

155,6 g 5-Methoxycarbonylmethoxy-IPSA werden mit 66 g 1-Methylamino-1-deoxy-D-arabit umgesetzt.

Die erhaltene Titelverbindung ist sehr gut löslich in Wasser (⩾ 100 % (g/v)).

DC : Rf = 0,55. Laufmittel $CHCl_3$/MeOH/$NH_4OH$=6 : 3 : 1

$[\alpha]_{365}^{20} = — 2{,}45°$ (c = 2 % in MeOH)

## Beispiel 26

R-5(1((N-Methyl-N-2,3,4,5-tetrahydroxypentyl) aminocarbonyl) ethoxy)-2,4,6-trijodisophthalsäure-bis(1,3-dihydroxyisopropylamid)

Formel I: $R_1=R_3=$ H; $R_2=R_4= -CH(CH_2OH)_2$; $R_5= -\overset{*}{C}HCONCH_2(CHOH)_3CH_2OH$; X = J
$\qquad\qquad\qquad\qquad\qquad\qquad\qquad\qquad\quad CH_3\ \ CH_3$

230 g R-5(1(Ethoxycarbonyl) ethoxy)-IPSA werden mit 121,6 g 1-Methylamino-1-deoxy-L-arabit umgesetzt.

Die erhaltene Titelverbindung schmilzt bei etwa 160-170 °C.

DC : Rf = 3,0. Laufmittel 2-Butanon/AcOH/Wasser=15 : 3 : 5.

$C_{23}H_{34}J_3N_3O_{12}$ : J ber. 41,15 % ; gef. 41,10 %;

$[\alpha]_{365}^{20} = + 10{,}4°$ (c = 2 % in MeOH)

Wasserlöslichkeit : ⩾ 100 % (g/v) bei 25 °C.

## Beispiel 27

5-(N-Methyl-N-2,3,4,5,6-pentahydroxyhexyl) aminocarbonylmethoxy-2,4,6-trijodisophthalsäure-bis(1,3-dihydroxyisopropylamid)

Formel I: $R_1=R_3=$ H; $R_2=R_4= -CH(CH_2OH)_2$; $R_5= -CH_2CONCH_2(CHOH)_4CH_2OH$;
X = J $\qquad\qquad\qquad\qquad\qquad\qquad\qquad CH_3$

23,3 g 5-Methoxycarbonylaminomethoxy-IPSA werden mit 11,7 g N-Methylglucamin (1-Methylamino-1-deoxy-D-glucitol) umgesetzt.

Die erhaltene amorphe Titelverbindung zerfliesst bei etwa 135 °C.

DC : Rf = 0,46. Laufmittel $CHCl_3$/MeOH/$NH_4OH$=2 : 2 : 1

$C_{23}H_{34}J_3N_3O_{13}$ : J ber. 40,44 % ; gef. 40,66 %;
$[\alpha]_{365}^{20}$ = — 8,61° (c = 2 % in MeOH)
Wasserlöslichkeit : 100 % (g/v) bei 20 °C.

Beispiel 28

R,S und R-5(1((N-Methyl-N-2,3,4,5,6-pentahydroxyhexyl) aminocarbonyl)-ethoxy)-2,4,6-trijodisophthalsäure-bis(1,3-dihydroxyisopropylamid)

```
Formel I: R1=R3= H; R2=R4= -CH(CH2OH)2; R5= -CHCONCH2(CHOH)4-CH2OH;
                                             |   |
          X = J                              CH3 CH3
```

Je 190 g 5(1(Ethoxycarbonyl) ethoxy)-IPSA (0,235 Mol) werden jeweils mit 105,6 g N-Methylglucamin umgesetzt.
Die erhaltene R,S-Titelverbindung schmilzt bei etwa 150 °C.
DC : Rf = 0,28. Laufmittel 2-Butanon/AcOH/$H_2O$=15 : 3 : 5
$C_{24}H_{36}J_3N_3O_{13}$ : J ber. 39,85 % ; gef. 39,60 %.
$[\alpha]_{365}^{20}$ = — 33,78° (c = 2 % in MeOH)
Wasserlöslichkeit : ≥ 100 % (g/v) bei 25 °C.
Die erhaltene R-Form der Titelverbindung schmilzt bei ca 140-145 °C.
DC : Rf = 0.28. $[\alpha]_{365}^{20}$ = + 12,65° (c = 2 % in MeOH) ; J gef. 39,49 %.
Wasserlöslichkeit : ≥ 100 % (g/v) bei 25 °C.

Beispiel 29

5(N-Methyl-N-2,3,4,5,6-pentahydroxyhexyl) aminocarbonylmethoxy-2,4,6-tribromisophthalsäure-bis(1,3-dihydroxyisopropylamid)

```
Formel I: R1=R3= H; R2=R4= -CH(CH2OH)2; R5= -CH2CONCH2(CHOH)4CH2OH;
                                                 |
          X = Br                                 CH3
```

15,9 g 5-Methoxycarbonylmethoxy-2,4,6-tribromisophthalsäure-bis(1,3-dihydroxyisopropylamid) werden mit 10 g N-Methylglucamin umgesetzt.
Die erhaltene Titelverbindung schmilzt bei 135-138 °C.
DC : Rf = 0,34. Laufmittel $CHCl_3$/MeOH/$NH_4OH$=2 : 2 : 2
$C_{23}H_{34}Br_3N_3O_{10}$ : Br ber. 29,95 % ; gef. 29,88 %.
Wasserlöslichkeit : ≥ 100 % (g/v) bei 20 °C.

Beispiel 30

5-Methylaminocarbonylmethoxy-2,4,6-trijodisophthalsäure-bis(2,3-dihydroxypropylamid)

Formel I : $R_1$=$R_3$=H ; $R_2$=$R_4$=—$CH_2CHOHCH_2OH$ ; $R_5$=—$CH_2CONHMe$ ; X=J

77 g 5-Methoxycarbonylmethoxy-2,4,6-trijodisophthalsäure-bis(2,3-dihydroxypropylamid) — abgekürzt 5-Methoxycarbonylmethoxy-IPDA — werden mit 50 ml 40 %igem wässrigem Methylamin bei 50 °C umgesetzt.
Die erhaltene Titelverbindung schmilzt bei 250 °C unter Zersetzung.
Wasserlöslichkeit : 2 % bei Seidetemperatur.
Ausgangsmaterial zu Zwischenprodukte :
5-Hydroxyisophthalsäure-bis(2,3-dihydroxypropylamid) :
105 g 5-Hydroxyisophthalsäuredimehtylester werden mit 182 g 1-Amino-2,3-propandiol 12 Stunden auf dem Dampfbad erhitzt. Der freigesetzte Methanol wird abdestilliert, das überschüssige Amin mittels Kationenaustauscher (Amberlite(R) IR-120 H(+)) entfernt.
Schmelzpunkt : 155 °C.
5-Hydroxy-2,4,6-trijodisophthalsäure-bis(2,3-dihydroxypropylamid) abgekürzt HIPDA :
328,3 g 5-Hydroxyisophthalsäure-bis(2,3-dihydroxypropylamid) (1 Mol) werden in 500 ml Wasser durch Zusatz von 10 N Natronlauge auf pH 9,4 gebracht und durch Eintropfen von 1 280 ml 2,5 molarer Kaliumjoddichlorid-Lösung unter Rühren jodiert.
Schmelzpunkt : 200 °C.
5-Methoxycarbonylmethoxy-2,4,6-trijodisophthalsäure-bis(2,3-dihydroxypropylamid) abgekürzt 5-Methoxycarbonylmethoxy-IPDA :

291,2 g Na-Salz von HIPDA werden in DMAC bei 80-200 °C mit 76,5 g Bromessigsäuremethylester umgesetzt. Salze werden mittels Ionenaustauscher entfernt. Das Produkt wird aus wenig Methanol umkristallisiert.

Schmelzpunkt : 230 °C.

Beispiel 31

5-(2-Hydroxyethylaminocarbonylmethoxy)-2,4,6-trijodisophthalsäure-bis(2,3-dihydroxypropylamid)

Formel I : $R_1=R_3=H$ ; $R_2=R_4=-CH_2CHOHCH_2OH$ ; $R_5=-CH_2CONHCH_2CH_2OH$ ; X=J

150 g 5-Methoxycarbonylmethoxy-IPDA werden durch Erhitzen mit 32 g 2-Hydroxyethylamin umgesetzt.

Schmelzpunkt : 250 °C unter Zersetzung.

DC : Rf = 0,52. Laufmittel 2-Butanon/AcOH/$H_2O$=15 : 3 : 5

Wasserlöslichkeit : 1,2 % bei 25 °C ; 13 % bei Siedetemperatur.

Beispiel 32

5-(2,3-Dihydroxypropylaminocarbonylmethoxy)-2,4,6-trijodisophthalsäure-bis(2,3-dihydroxypropylamid)

Formel I : $R_1=R_3=H$ ; $R_2=R_4=-CH_2CHOHCH_2OH$ ; $R_5=-CH_2CONHCH_2CHOHCH_2OH$ ; X=J

113 g 5-Methoxycarbonylmethoxy-IPDA werden mit 27,3 g 1-Amino-2,3-propandiol umgesetzt.

Schmelzpunkt : 250 °C unter Zersetzung.

DC : Rf=0,51. Laufmittel $CHCl_3$/MeOH/$NH_4OH$=2 : 2 : 1

Wasserlöslichkeit : 1 % bei 20 °C, 10 % bei Siedetemperatur.

Beispiel 33

5-(1,3-Dihydroxy-2-methylisopropylaminocarbonylmethoxy)-2,4,6-trijodisophthalsäure-bis(2,3-dihydroxypropylamid)

Formel I : $R_1=R_3=H$ ; $R_2=R_4=-CH_2CHOHCH_2OH$ ; $R_5=-CH_2CONHC(CH_3)(CH_2OH)_2$ ; X=J

155,6 g 5-Methoxycarbonylmethoxy-IPDA werden mit 105 g 1,1-Bis(hydroxymethyl) ethylamin (= 2-Methyl-2-amino-1,3-propandiol) umgesetzt.

Schmelzpunkt der erhaltenen Titelverbindung : 170 °C

$C_{20}H_{28}J_3N_3O_{10}$ : J ber. 44,73 % ; gef. 44,72 %.

Wasserlöslichkeit : ≥ 100 % (g/v) bei 25 °C.

Beispiel 34

5-(N-Methyl-N-2,3-dihydroxypropyl) aminocarbonylmethoxy-2,4,6-trijodisophthalsäure-bis(2,3-dihydroxypropylamid)

Formel I: $R_1=R_3=$ H; $R_2=R_4=$ $-CH_2CHOHCH_2OH$; $R_5=$ $-CH_2CON(CH_3)CH_2CHOHCH_2OH$;
X = J

115 g 5-Methoxycarbonylmethoxy-IPDA und 58,3 g 1-Methylamino-2,3-propandiol werden in einem niedrigen Alkohol durch Kochen am Rückflusskühler umgesetzt.

Die dabei erhaltene Titelverbindung schmilzt bei etwa 150 °C

DC : Rf = 0,3. Laufmittel 2-Butanon/AcOH/$H_2O$ = 15 : 3 : 5

Wasserlöslichkeit : ≥ 100 % (g/v) bei 25 °C.

Beispiel 35

5-(N-Methyl-N-2,3,4-trihydroxybutyl) aminocarbonylmethoxy-2,4,6-trijodisophthalsäure-bis(2,3-dihydroxypropylamid)

Formel I: $R_1=R_3=$ H; $R_2=R_4=$ $-CH_2CHOHCH_2OH$; $R_5=$ $-CH_2CON(CH_3)CH(CHOH)_2CH_2OH$;
X = J

115,6 g 5-Methoxycarbonylmethoxy-IPDA werden mit 67,6 g 1-Methylamino-1-deoxy-D-erythrol umgesetzt.

DC : Rf = 0,36. Laufmittel $CHCl_3/MeOH/NH_4OH = 2:2:1$

$[\alpha]_{365}^{20} = -4{,}6°$ (c = 2 % in MeOH)

Wasserlöslichkeit : ≥ 100 % (g/v) bei 25 °C.

Beispiel 36

5-(N-Methyl-N-2,3,4,5-tetrahydroxypentyl) aminocarbonylmethoxy-2,4,6-trijodisophthalsäure-bis(2,3-dihydroxypropylamid)

Formel I: $R_1=R_3=$ H; $R_2=R_4=$ $-CH_2CHOHCH_2OH$; $R_5=$ $-CH_2CON(CH_3)CH_2(CHOH)_3CH_2OH$;
X = J

111 g 5-Methoxycarbonylmethoxy-IPDA werden mit 59 g 1-Methylamino-1-deoxy-L-arabit umgesetzt.

DC : Rf = 0,39. Laufmittel $CHCl_3/MeOH/NH_4OH = 2:2:1$

$[\alpha]_{365}^{20} = +8{,}33°$ (c = 2 % in MeOH)

Wasserlöslichkeit : ≥ 100 % (g/v) bei 25 °C.

Beispiel 37

5-(N-Methyl-N-2,3,4,5,6-pentahydroxyhexyl) aminocarbonylmethoxy-2,4,6-trijodisophthalsäure-bis(2,3-dihydroxypropylamid)

Formel I: $R_1=R_3=$ H; $R_2=R_4=$ $-CH_2CHOHCH_2OH$; $R_5=$ $-CH_2CON(CH_3)CH_2(CHOH)_4CH_2OH$;
X = J

217 g 5-Methoxycarbonylmethoxy-IPDA werden mit 108 g N-Methylglucamin umgesetzt.

Schmelzpunkt der erhaltenen Titelverbindung : ~ 150 °C.

$[\alpha]_{365}^{20} = -7{,}9°$ (c = 2 % in MeOH)

Wasserlöslichkeit : ≥ 100 % (g/v) bei 20 °C.

Beispiel 38

5-(N-Methyl-N-2,3,4,5,6-pentahydroxyhexyl) aminocarbonylmethoxy-2,4,6-tribromisophthalsäure-bis(2,3-dihydroxypropylamid)

Formel I: $R_1=R_3=$ H; $R_2=R_4=$ $-CH_2CHOHCH_2OH$; $R_5=$ $-CH_2CON(CH_3)CH_2(CHOH)_4CH_2OH$;
X = Br

95,5 g 5-Methoxycarbonylmethoxy-2,4,6-tribromisophthalsäure-bis(2,3-dihydroxypropylamid) werden mit 58,5 g N-Methylglucamin umgesetzt.

DC : Rf = 0,27. Laufmittel $AcOEt/AcOH/H_2O = 20:10:6$

$[\alpha]_{365}^{20} = -11{,}9°$ (c = 2 % in MeOH)

Wasserlöslichkeit : ≥ 100 % (g/v) bei 20 °C.

Ausgangsmaterial und Zwischenprodukt :

5-Hydroxy-2,4,6-tribromisophthalsäure-bis(2,3-dihydroxypropylamid) :

218 g 5-Hydroxy-2,4,6-tribromisophthalsäuredichlorid (siehe Beispiel 78) werden in einem inerten Lösungsmittel mit 240 g 1-Amino-2,3-propandiol umgesetzt.

Erhaltenes Produkt schmilzt nach Umkristallisieren aus Ethanol bei 222 °C.

5-Methoxycarbonylmethoxy-2,4,6-tribromisophthalsäure-bis(2,3-dihydroxypropylamid) :

176 g Na-Salz von 5-Hydroxy-2,4,6-tribromisophthalsäure-bis(2,3-dihydroxypropylamid) werden mit 50 g Bromessigsäuremethylester umgesetzt.

Erhaltenes Produkt schmilzt bei ~ 150 °C.

Beispiel 39

5(N-Methyl-N-2,3-dihydroxypropyl)aminocarbonylmethoxy-2,4,6-trijodisophthalsäure-bis(1,3-dihydroxy-2-methylisopropylamid)

Formel I: $R_1=R_3=H$; $R_2=R_4=-C(CH_3)(CH_2OH)_2$; $R_5=-CH_2CON(CH_3)CH_2CHOHCH_2OH$;
$X=J$

42,7 g 5-Methoxycarbonylmethoxy-2,4,6-trijodisophthalsäure-bis(1,3-dihydroxy-2-methyl-isopropylamid) werden mit 21 g 1-Methylamino-2,3-propandiol umgesetzt.

Die erhaltene Titelverbindung schmilzt bei 205-210 °C.

DC : Rf = 0,35. Laufmittel $CHCl_3/MeOH/NH_4OH = 6:3:1$

$C_{22}H_{32}J_3N_3O_{10}$ : J ber. 43,30 % ; gef. 43,28 %

Wasserlöslichkeit : ≥ 100 % (g/v) bei 20 °C.

Ausgangsmaterial und Zwischenprodukt :

5-Hydroxy-2,4,6-trijodisophthalsäure-bis(1,3-dihydroxy-2-methylisopropylamid) :

59,7 g 5-Hydroxy-2,4,6-trijodisophthalsäuredichlorid (0,1 Mol) werden mit 52,6 g 2-Amino-2-methyl-1,3-propandiol (0,5 Mol) umgesetzt.

$C_{16}H_{21}J_3N_2O_7$ : J ber. 51,88 % ; gef. 51,81 %.

5-Methoxycarbonylmethoxy-2,4,6-trijodisophthalsäure-bis(1,3-dihydroxy-2-methylisopropylamid) :
57 g Na-Salz von 5-Hydroxy-2,4,6-trijodisophthalsäure-bis(1,3-dihydroxy-2-methyl-isopropylamid) werden mit 15,3 g Bromessigsäuremethylester umgesetzt.

Schmelzpunkt : 170 °C.

## Beispiel 40

5-(3,6-Dioxaoctyl) oxy-2,4,6-trijodisophthalsäure-bis(N-methyl-N-2,3-dihydroxypropylamid)

Formel I : $R_1 = R_3 = CH_3$ ; $R_2 = R_4 = -CH_2CHOHCH_2OH$ ; $R_5 = -(CH_2CH_2O)_2-C_2H_5$ ; X = J

57 g Na-Salz von 5-Hydroxy-2,4,6-trijodisophthalsäure-bis(N-methyl-N-2,3-dihydroxypropylamid) (0,07 Mol) werden in DMAC mit 23,2 g 1(4-Nitrobenzolsulfonyloxy)-3,6-dioxaoctan (0,07 Mol) analog Beispiel 1 umgesetzt.

Die erhaltene Titelverbindung ist sehr gut wasserlöslich (≥ 60 % (g/v) bei 25 °C).

5-Hydroxy-2,4,6-trijodisophthalsäure-bis(N-methyl-N-2,3-dihydroxypropylamid) wird erhalten durch Umsetzung von 57,9 g 5-Hydroxy-2,4,6-trijodisophthalsäuredichlorid mit 52 g 1-Methylamino-2,3-propandiol.

## Beispiel 41

5-(3,6-Dioxaoctyl) oxy-2,4,6-trijodisophthalsäure-bis(N-2-hydroxyethyl-N-2,3-dihydroxypropylamid)

Formel I : $R_1 = R_2 = -CH_2CH_2OH$ ; $R_3 = R_4 = -CH_2CHOHCH_2OH$ ; $R_5 = -(CH_2CH_2O)_2C_2H_5$ ; X = J

55,6 g Na-Salz von 5-Hydroxy-2,4,6-trijodisophthalsäure-bis(N-2-hydroxyethyl-N-2,3-dihydroxypropylamid) werden in DMAC mit 23,2 g 1(4-Nitrobenzolsulfonyloxy)-3,6-dioxaoctan umgesetzt.

Die erhaltene Titelverbindung ist selbst bei Raumtemperatur praktisch unbeschränkt löslich in Wasser : ≥ 100 % (g/v).

5-Hydroxy-2,4,6-trijodisophthalsäure-bis(N-2-hydroxyethyl-N-2,3-dihydroxyproylamid) wird erhalten durch Umsetzung von 59,7 g 5-Hydroxy-2,4,6-trijodisophthalsäuredichlorid mit 120,5 g N-2-Hydroxyethyl-N-2,3-dihydroxypropylamin.

## Beispiel 42

1-(3-(1,3-Dihydroxyisopropylaminocarbonyl)-5-(2,3-dihydroxypropylaminocarbonyl)-2,4,6-trijod-phenoxy)-3,6-dioxaoctan

Formel I : $R_1 = R_3 = H$ ; $R_2 = -CH(CH_2OH)_2$ ; $R_4 = -CH_2CHOHCH_2OH$ ; $R_5 = -(CH_2CH_2O)_2CH_2CH_2-$ ; X = J

62,5 g Na-Salz von 3-(1,3-Dihydroxyisopropylaminocarbonyl)-5-(2,3-dihydroxypropylaminocarbonyl)-2,4,6-trijodphenol werden mit 26,5 g 1(4-Nitrobenzolsulfonyloxy)-3,6-dioxaoctan umgesetzt.

Die erhaltene Titelverbindung ist sehr gut wasserlöslich : ≥ 50 % (g/v). 3-(1,3-Dihydroxyisopropylaminocarbonyl)-5-(2,3-dihydroxypropylaminocarbonyl)-2,4,6-trijodphenol wird erhalten, wenn man 56 g 5-Hydroxy-2,4,6-trijodisophthalsäuredichlorid zunächst mit 18,2 g Serinol und danach mit 18,2 g 1-Amino-2,3-dihydroxypropylamin umsetzt.

## Beispiel 43

1,8-Bis-(3,5-bis(1,3-dihydroxyisopropylaminocarbonyl)-2,4,6-trijodphenoxy)-3,6-dioxaoctan

Formel I : $R_1 = R_2 = H$ ; $R_2 = R_4 = —CH(CH_2OH)_2$ ; $R_5 = —(CH_2CH_2O)_2CH_2CH_2—$ ; X = J

58 g Na-Salz von HIPSA (0,08 Mol) werden in 300 ml DMAC mit 1,8-Bis-(4-nitrobenzolsulfonyloxy)-3,6-dioxacotan bei 70 °C umgesetzt.

Schmelzpunkt : 280 °C unter Zersetzung.

$C_{34}H_{44}J_6N_4O_{16}$ : J ber. 49,88 % ; gef. 50,02 %.

Wenig löslich in Wasser.

1,8-Bis(4-nitrobenzolsulfonyloxy)-3,6-dioxaoctan wird erhalten durch Umsetzung von 15 g Triethylenglykol (0,1 Mol) mit 42 g 4-Nitrobenzolsulfonylchlorid in Gegenwart von 28 g Triethylamin (0,2 Mol) in Ethylacetat.

Schmelzpunkt : 101-102 °C.

## Beispiel 44

1,8-Bis-(3,5-bis(2,3-dihydroxypropylaminocarbonyl)-2,4,6-trijodphenoxy)-3,6-dioxan

Formel I : $R_1 = R_3 = H$ ; $R_2 = R_4 = —CH_2CHOHCH_2OH$ ; $R_5 = —(CH_2CH_2O)_2CH_2CH_2—$ ; X = J

141 g Na-Salz von 5-Hydroxy-2,4,6-trijodisophthalsäure-bis(2,2-dimethyl-1,3-dioxolan-4-yl-methylamid (0,174 Mol) in 500 ml DMAC werden unter Rühren mit 45,8 g 1,8-Bis-(4-nitrobenzolsulfonyloxy)-3,6-dioxan (0,087 Mol) bei etwa 70 °C umgesetzt. Die Reaktionslösung wird im Vakuum konzentriert und in Wasser eingerührt.

Man erhält 1,8-Bis-(3,5-bis(2,2-dimethyl-1,3-dioxolan-4-yl-methylaminocarbonyl)-2,4,6-trijodphenoxy)-3,6-dioxan vom Schmelzpunkt ca 262 °C.

DC : Rf 0,21. Laufmittel $AcOBu/AcOH/H_2O$ = 5 : 1 : 1.

80 g (0,047 5 Mol) des obigen Zwischenproduktes mit durch Ketalisierung geschützten Hydroxy-Funktionen suspendiert in 1 200 ml 80 %igem wässrigem Dioxan werden unter Rühren mit 10 ml 18 %iger wässriger Salzsäure versetzt. Die entstandene Lösung wird mit Natronlauge neutralisiert und zur Trockene verdampft.

Die erhaltene Titelverbindung schmilzt bei ca 270 °C.

DC : Rf 0,1. Laufmittel $CHCl_3/MeOH/NH_4OH$ = 6 : 3 : 1

$C_{34}H_{44}J_6N_4O_{16}$ : J ber. 49,89 % : gef. 49,70 %.

Wenig löslich in Wasser.

Herstellung von 5-Hydroxy-2,4,6-trijodisophthalsäure-bis(2,2-dimethyl-1,3-dioxolan-4-yl-methylamid) :

131,2 g 2,2-Dimethyl-4-aminomethyl-1,3-dioxolan werden in 500 ml DMAC mit 119,5 g 5-Hydroxy-2,4,6-trijodisophthalsäuredichlorid umgesetzt. Schmelzpunkt : ~ 150 °C ; DC : Rf 0,57 mit $CHCl_3/MeOH/NH_4OH$ = 6 : 3 : 1.

## Beispiel 45

1,14-Bis-(3-(1,3-dihydroxyisopropylaminocarbonyl)-5-(2,3-dihydroxypropylaminocarbonyl)-2,4,6-trijodphenoxy)-3,6,9,12-tetraoxatetradecan

Formel I : $R_1 = R_3 = H$ ; $R_2 = —CH(CH_2OH)_2$ ; $R_4 = —CH_2CHOHCH_2OH$ ; $R_5 = —(CH_2CH_2O)_4CH_2CH_2—$, X = J

Diese Verbindung wird erhalten durch Umsetzung von 3-(1,3-Dihydroxyisopropylaminocarbonyl)-5-(2,3-dihydroxypropylaminocarbonyl)-2,4,6-trijodphenol mit 1,14-Bis(4-nitrobenzolsulfonyloxy)-3,6,9,12-tetraoxatetradecan.

Die erhaltene Titelverbindung ist gut wasserlöslich.

## Beispiel 46

1,2-Bis-(3,5-bis(1,3-dihydroxyisopropylaminocarbonyl)-2,4,6-trijodphenoxy-acetylamino)-ethan

Formel I : $R_1 = R_3 = H$ ; $R_2 = R_4 = —CH(CH_2OH)_2$ ; $R_5 = —CH_2CONHCH_2CH_2NHCOCH_2—$ ; X = J

Diese Verbindung wird erhalten durch Umsetzung des Na-Salzes von HIPSA mit 1,2-Bis-(bromacetylamino) ethan.

Die Titelverbindung ist kaum löslich in Wasser.

Beispiel 47

1,4-Bis-(3,5-bis(1,3-dihydroxyisopropylaminocarbonyl)-2,4,6-trijodphenoxy-acetylamino)-2,3-dihydroxybutan

Formel I : $R_1 = R_3 = H$ ;
$R_2 = R_4 = —CH(CH_2OH)_2$ ; $R_5 = —CH_2CONHCH_2CHOH—CHOHCH_2NHCOCH_2—$, X = J

Diese Verbindung wird erhalten durch Umsetzung des Na-Salzes von HIPSA mit 1,4-Bis-(chloracetylamino)-2,3-dihydroxybutan.

Die Titelverbindung ist wasserlöslich.

Beispiel 48

5-(1,3-Dihydroxyisopropylaminocarbonyl) methoxy-2,4,6-trijodisophthalsäure-bis(2,3-dihydroxypropylamid)

Formel I : $R_1 = R_3 = H$ ; $R_2 = R_4 = —CH_2CHOHCH_2OH$ ; $R_5 = —CH_2CONHCH(CH_2OH)_2$ ; X = J

87 g 5-Hydroxy-2,4,6-trijodisophthalsäure-bis(2,2-dimethyl-1,3-dioxolan-4-yl-methylamid) werden in 100 ml Aceton mit 22 g Chloressigsäure-(1,3-dihydroxyisopropylamid) in Gegenwart von 28 g fein gepulvertem, trockenem Kaliumcarbonat und 5 g Natriumjodid am Rückfluss gerührt und in üblicher Weise aufgearbeitet.

Das erhaltene 5-(1,3-Dihydroxyisopropylaminocarbonyl) methoxy-2,4,6-trijodisophthalsäure-bis(2,2-dimethyl-1,3-dioxolan-4-yl-methylamid) wird in Methanol mit 5 N wässriger Salzsäure versetzt, nach einigen Stunden neutralisiert, danach durch Ionenaustauscher entsalzt und wie üblich aufgearbeitet. Die erhaltene Titelverbindung ist leicht löslich in Wasser.

Beispiel 49

5-(2,3-Dihydroxypropylaminocarbonyl) methoxy-2,4,6-trijodisophthalsäure-bis(1,3-dihydroxyisopropylamid)

Formel I : $R_1 = R_3 = H$ ; $R_2 = R_4 = —CH(CH_2OH)_2$ ; $R_5 = —CH_2CONHCH_2CHOHCH_2OH$ ; X = J

157 g 5-Hydroxy-2,4,6-trijodisophthalsäure-bis(2,2-dimethyl-1,3-dioxan-5-yl-amid (0,2 Mol) werden mit 70 g 5-(Bromacetyl)amino-2,2-dimethyl-1,3-dioxan in 200 ml Aceton in Gegenwart von 56 g Kaliumcarbonat und 5 g Natriumjodid unter Rühren bei Siedetemperatur umgesetzt.

Das erhaltene 5-(2,2-Dimethyl-1,3-dioxolan-4-yl-methylaminocarbonyl) methoxy-2,4,6-trijodisophthalsäure-bis(2,2-dimethyl-1,3-dioxan-5-yl-amid) wird in Tetrahydrofuran (THF) mit 5 N Salzsäure versetzt, nach 2 Tagen neutralisiert und aufgearbeitet.

Die erhaltene Titelverbindung ist leicht löslich in Wasser.

5-Hydroxy-2,4,6-trijodisophthalsäure-bis(2,2-dimethyl-1,3-dioxan-5-yl-amid wird erhalten durch Umsetzung von 179,1 g 5-Hydroxy-2,4,6-trijodisophthalsäure-dichlorid mit 216,3 g 5-Amino-2,2-dimethyl-1,3-dioxan in THF.

Schmelzpunkt : 170 °C unter Zersetzung.

Beispiel 50

5-Hexadecyloxy-2,4,6-trijodisophthalsäure-bis(1,3-dihydroxyisoproylamid)

Formel I : $R_1 = R_3 = H$ ; $R_2 = R_4 = —CH(CH_2OH)_2$ ; $R_5 = —C_{16}H_{33}$ ; X = J

145 g Na-HIPSA werden bei 60 °C in 500 ml DMAC mit 70 g 1-Bromhexadecan umgesetzt. Das Produkt wird aus Methanol umkristallisiert.

Die erhaltene Titelverbindung schmilzt bei 228 °C.

DC : RF = 0,56. Laufmittel $CHCl_3/MeOH/NH_4OH = 6 : 3 : 1$

Beispiel 51

5-Hexadecyloxy-2,4,6-trijodisophthalsäure-bis(2,3-dihydroxypropylamid)

Formel I : $R_1 = R_3 = H$ ; $R_2 = R_4 = —CH_2CH(OH)CH_2OH$ ; $R_5 = —C_{16}H_{33}$ ; X = J

75 g Na-Salz von HIPDA werden bei 60 °C in 250 ml DMAC mit 40 g 1-Bromhexadecan umgesetzt. Das Produkt wird aus Methanol umkristallisiert.
Schmelzpunkt : 245 °C
DC : Rf = 0,6. Laufmittel $CHCl_3/MeOH/NH_4OH$ = 6 : 3 : 1

Verwendung

Die vorstehend beschriebenen Verbindungen sind hervorragend geeignet als schattengebende Komponenten in Röntgenkontrastmitteln.

Die in Wasser sehr gut löslichen Verbindungen werden zu Röntgenkontrastmitteln für die Vasographie, Urographie, zur Darstellung von Körperhöhlen und der Liquorräume sowie für gewisse Lymphographien (etwa der Extremitäten) verwendet.

Voraussetzung für diese Anwendungen sind neben sehr hoher Wasserlöslichkeit insbesondere höchste Verträglichkeit und genügende Stabilität. Verträglichkeit :

Bei drei typischen Verbindungen der vorliegenden Erfindung sind die auf der nachfolgenden Tabelle aufgeführten Toxizitätsdaten gemessen worden.

Code B 18580 :
5-(8-Hydroxy-3,6-dioxaoctyl) oxy-2,4,6-trijodisophthalsäure-bis(1,3-dihydroxyisopropylamid) ;
Code B 18570 :
5-(3,5,8-Trioxadecyl) oxy-2,4,6-trijod-isophthalsäure-bis(1,3-dihydroxyisopropylamid) ;
Code B 18260 :
5-(N-Methyl-N-2,3-dihydroxypropyl) aminocarbonylmethoxy-2,4,6-trijodisophthalsäure-bis(1,3-dihydroxyisopropylamid).

Tabelle I

| Verbindung | Toxizität: Dosis letalis 50 % (DL 50) bei der Maus | | | |
|---|---|---|---|---|
| | intravenös | | intracerebral | |
| | mg Verbg/kg | mg J/kg | mg Verbg/kg | mg J/kg |
| B 18580 | 27'740 | 12'600 | 2'400 | 1'090 |
| B 18570 | 11'190 | 5'000 | 2'395 | 1'070 |
| B 18260 | 29'754 | 13'300 | 2'097 | 920 |

Damit ist bewiesen, dass diese Verbindungsklasse hervorragend verträglich ist.

Injektionslösungen

Je nach Verwendungszweck kommen ca 15-85 %ige wässrige Lösungen mit einem Gehalt von etwa 50 bis 500, vorzugsweise von 100 bis 400 mg J/ml zur Anwendung. Konzentrierte Lösungen werden bevorzugt. Die Art der Applikation richtet sich nach dem sichtbar zu machenden Organ.

Es wurden Kontrastmittelmengen appliziert, die bis zu 80 g Jod enthalten.

Für die Vasographie werden die Lösungen in die entsprechenden Blutgefässe injiziert oder infundiert.

Für die Urographie werden die Lösungen intravenös injiziert oder infundiert.

Für die Kontrastverstärkung in der Computertomographie werden die Lösungen je nach zu verstärkendem Organ- oder Gewebekontrast entweder durch intravenöse Verabreichung in den Blutkreislauf eingeführt oder durch selektive Injektion im Gefässsystem eines einzelnen Organs oder in einer Körperhöhle angereichert.

Für die Myelographie und Radiculographie werden die Lösungen nach lumbaler oder subokzipitaler Punktion instilliert.

Dosierung

Myelographie ca 5-15 ml) bei einem Jodgehalt von
Radiculographie ca 3-5 ml) 200 mg J/ml

Die Herstellung der Röntgenkontrastmittellösungen ist einfach, weil keine Salzlösungen bereitet werden müssen.

Beispielsweise werden die nach den vorstehenden Beispielen erhaltenen reinen 5-Alkoxy-2,4,6-trijodisopthalsäurediamide unter sterilen Bedingungen in der gewünschten Menge bidestilliertem Wasser gelöst, filtriert, unter Zusatz von geringen Mengen EDTA-$Na_2$ und Puffersubstanzen auf pH 6,9 bis 7,1 eingestellt, in Serumflaschen oder Ampullen abgefüllt und anschliessend sterilisiert.

Beispiele 52-60

Typische Beispiele für die Zusammensetzung von Injektionslösungen :

Verbindungen

Code B 18580/B 18570/B 18260 ;
Code B 18600
5-(3,6,9,12,15,18,21-Heptaoxadocosyl) oxy-2,4,6-trijodisophthalsäure-bis(1,3-dihydroxyisopropylamid) ;
Code B 18630
5-(3,6,9,12,15-Pentaoxahexadecyl) oxy-2,4,6-trijodisophthalsäure-bis-(1,3-dihydroxyisopropylamid) ;
Code B 18610
5-(Bis(2-hydroxyethyl) aminocarbonylmethoxy)-2,4,6-trijodisophthalsäure-bis(1,3-dihydroxyisopropylamid) ;
Code B 18620
R,S-5-(1-(Bis(2-hydroxyethyl) aminocarbonyl) ethoxy)-2,4,6-trijodisophtalsäure-bis(1,3-dihydroxyisopropylamid) ;
Code B 18500
5-(1,3-Dihydroxy-2-methylisopropylaminocarbonyl) methoxy-2,4,6-trijodisophthalsäure-bis(2,3-dihydroxypropylamid) ;
Code B 18720
5-((N-Methyl-N-2,3-dihydroxypropyl) aminocarbonyl) methoxy-2,4,6-trijodisophthalsäure-bis(1,3-dihydroxy-2-methylisopropylamid).

Zlusammensetzung von Injektionslösungen enthaltend 200, 300 und 400 mg J/ml.

Injektionslösungen mit 200 mg J/ml enthalten :

x (siehe Tabelle) g Jodverbindung, 0,22 g Di-Na-Salz von Ethylendiamintetraessigsäure dihydrat (EDTA-$Na_2 \cdot 2\ H_2O$), etwa 0,7 g Tris-(hydroxymethyl) aminomethan (Tromethamin), 0,5 g Tromethamin-hydrochlorid und bidestilliertes Wasser bis zum Totalvolumen der Lösung von 1 000 ml.

Injektionslösungen mit 300 mg J/ml enthalten :

y (siehe Tabelle) g Jodverbindung, 0,3 g EDTA-$Na_2 \cdot 2\ H_2O$, etwa 1 g Tromethamin, 0,8 g Tromethamin-hydrochlorid und bidestilliertes Wasser ad 1 000 ml.

Injektionslösungen mit 400 mg J/ml enthalten :

z (siehe Tabelle) g Jodverbindung, 0,5 g EDTA-$Na_2 \cdot 2\ H_2O$, etwa 1,5 g Tromethamin, 1 g Tromethamin-hydrochlorid und bidestilliertes Wasser ad 1 000 ml.

Tabelle für die benötigten Mengen der individuellen Jod-Verbindungen

| Verbindung (Code) | x g (200 mg J/ml) | y g (300 mg J/ml) | z g (400 mg J/ml) |
|---|---|---|---|
| B 18580 | 440 | 660 | 880 |
| B 18570 | 447 | 671 | 895 |
| B 18600 | 540 | 810 | 1'080 |
| B 18630 | 494 | 741 | 980 |
| B 18610 | 448 | 672 | 896 |
| B 18620 | 458 | 687 | 916 |
| B 18260 | 447 | 671 | 895 |
| B 18500 | 447 | 671 | 895 |
| B 18720 | 462 | 693 | 924 |

Die Injektionslösungen von B 18580, B 18570, B 18600, B 18630 lassen sich ohne Schwierigkeiten sterilisieren durch Erhitzen auf 120 °C während 30 Minuten.

Die Injektionslösungen von B 18610, B 18620, B 18260, B 18500 und B 18720 werden zweckmässig durch Ultrafiltration sterilisiert.

Die wässrigen Lösungen der Brom-Verbindungen wie etwa diejenige von 5-(2,5,8-Trioxadecyl) oxy-2,4,6-tribromisophthalsäure-bis(1,3-dihydroxyisopropylamid) (Code B 18820) lassen sich ohne Schwierigkeiten durch Erhitzen (30'/120 °C) sterilisieren.

Die in Wasser wenig löslichen einschlägigen 5-Alkoxy-2,4,6-trihalogenisophthalsäurediamide der Formel I eignen sich zur Herstellung von gut verträglichen Suspensionen, Liposomen oder ultrafeinen Präparaten mit Partikeln im Mikro- und Nanometerbereich für die Lymphographie, Hepatographie, Splenographie, Hysterosalpingographie, Bronchographie sowie die Darstellung anderer Körperhöhlen.

**Patentansprüche** (für die Vertragsstaaten : BE, CH, DE, FR, GB, IT, LI, LU, NL, SE)

1. 5-Alkoxy-2,4,6-trijod- bzw. tribrom-isophthalsäure-diamide der allgemeinen Formel (I)

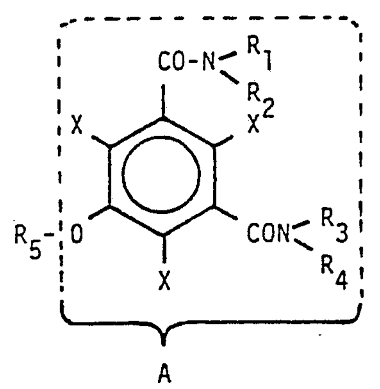

(I)

worin

X Jod oder Brom

$R_1$ und $R_3$ gleich oder verschieden sein können, nämlich Wasserstoff, Niederalkyl, Hydroxyalkyl,

$R_2$ und $R_4$ gleich oder verschieden sein können, nämlich Hydroxyalkyl mit 1 bis 5 HO-Funktionen und 2 bis 6 C-Atomen,

$R_5$ Hydroxyalkyl mit 1 bis 5 HO-Funktionen und 2 bis 6 C-Atomen, (Poly) Alkoxyalkyl mit 1 bis 10 Oxa-Funktionen und 3 bis 33 C-Atomen, Hydroxy(poly) alkyl mit 1 bis 10 Oxa-Funktionen und 4 bis 33 C-Atomen Aminocarbonylalkyl der Formel

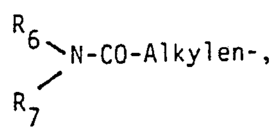

worin

$R_6$ H, Alkyl oder Hydroxyalkyl

$R_7$ Hydroxyalkyl mit 1 bis 5 HO-Funktionen und 2 bis 6 C-Atomen und

Alkylen gerades oder verzweigtes zweiwertiges Alkylen mit 1 bis 5 C-Atomen bedeutet,

$R_5$ Alkyl mit 10 bis 20 C-Atomen oder A-Alkylen'-, worin Alkylen' zweiwertiges Alkylen mit 2 bis 12 C-Atomen mit 0 bis 5 Oxa-, Thia-, HO- oder Amid-Funktionen und A einen 3,5-Bis-(aminocarbonyl)-2,4,6-trihalogen-phenoxy-Rest der Formel I darstellt, der sich innerhalb der gestrichelten Linie befindet, bedeutet.

2. Röntgenkontrastmittel enthaltend eine schattengebende Substanz gemäss Patentanspruch 1.

3. Verfahren zur Herstellung von Verbindungen der allgemeinen Formel I, dadurch gekennzeichnet, dass man in an sich bekannter Weise eine Verbindung der allgemeinen Formel II

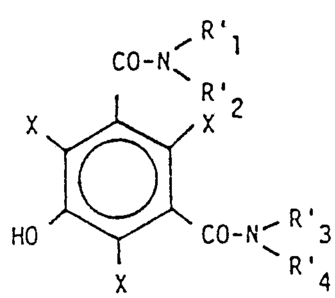

(II)

worin X, $R'_1$, $R'_2$, $R'_3$ und $R'_4$ die weiter oben genannte Bedeutung für X, $R_1$, $R_2$, $R_3$ und $R_4$ besitzt, aber im Molekül vorhandene freie Hydroxylgruppen auch in geschützter Form vorliegen können, bzw. ein Alkali- oder Erdalkalisalz davon mit einer Verbindung der allgemeinen Formel III

$$R'_5\text{—}Z \qquad (III)$$

umsetzt, worin Z ein reaktives Halogenatom Cl, Br oder Jod oder ein reaktiver Sulfosäurerest bedeutet und $R'_5$ die weiter oben genannte Bedeutung für $R_5$ besitzt oder eine Alkoxycarbonylalkyl-Gruppe mit 3 bis 8 C-Atomen darstellt, und in der erhaltenen Verbindung der Formel IV

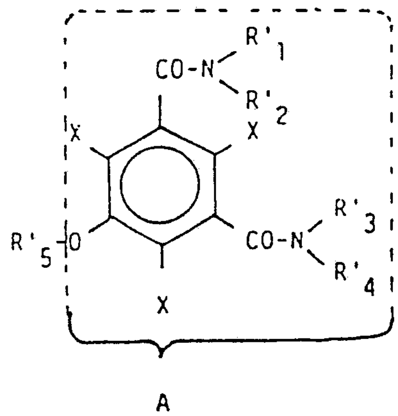

(IV)

worin die verschiedenen Symbole die weiter oben genannte Bedeutung besitzen, vorhandene 5-Alkoxycarbonylalkoxy-Gruppen durch Umsetzung mit einem primären und sekundären Amin in die entsprechende 5-Aminocarbonylalkoxy-Gruppe überführt und schliesslich Hydroxy-Funktionen maskierende Schutzgruppen durch Hydrolyse entfernt und die Verbindung der Formel I isoliert.

**Patentanspruch** (für den Vertragsstaat AT)

Verfahren zur Herstellung von 5-Alkoxy-2,4,6-trijod- bzw. tribrom-isophthalsäure-diamide der allgemeinen Formel (I)

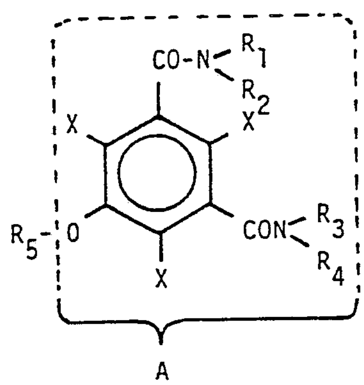

(I)

worin

X Jod oder Brom

$R_1$ und $R_3$ gleich oder verschieden sein können, nämlich Wasserstoff, Niederalkyl, Hydroxyalkyl,

$R_2$ und $R_4$ gleich oder verschieden sein können, nämlich Hydroxyalkyl mit 1 bis 5 HO-Funktionen und 2 bis 6 C-Atomen,

$R_5$ Hydroxyalkyl mit 1 bis 5 HO-Funktionen und 2 bis 6 C-Atomen, (Poly)Alkoxyalkyl mit 1 bis 10 Oxa-Funktionen und 3 bis 33 C-Atomen, Hydroxy(poly)alkyl mit 1 bis 10 Oxa-Funktionen und 4 bis 33 C-Atomen Aminocarbonylalkyl der Formel

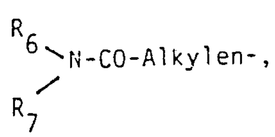

worin

$R_6$ H, Alkyl oder Hydroxyalkyl

$R_7$ Hydroxyalkyl mit 1 bis 5 HO-Funktionen und 2 bis 6 C-Atomen und

Alkylen gerades oder verzweigtes zweiwertiges Alkylen mit 1 bis 5 C-Atomen bedeutet,

$R_5$ Alkyl mit 10 bis 20 C-Atomen oder A-Alkylen'-, worin Alkylen' zweiwertiges Alkylen mit 2 bis 12 C-Atomen mit 0 bis 5 Oxa-, Thia-, HO- oder Amid-Funktionen und A einen 3,5-Bis-(aminocarbonyl)-2,4,6-trihalogen-phenoxy-Rest der Formel I darstellt, der sich innerhalb der gestrichelten Linie befindet, bedeutet, dadurch gekennzeichnet, dass man in an sich bekannter Weise eine Verbindung der allgemeinen Formel II

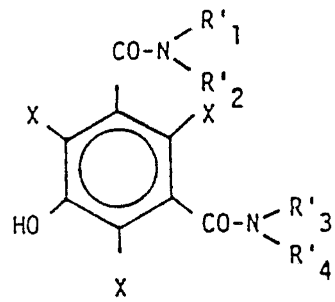

(II)

worin X, $R'_1$, $R'_2$, $R'_3$ und $R'_4$ die weiter oben genannte Bedeutung für X, $R_1$, $R_2$ $R_3$ und $R_4$ besitzt, aber im Molekül vorhandene freie Hydroxylgruppen auch in geschützter Form vorliegen können, bzw. ein Alkali- oder Erdalkalisalz davon mit einer Verbindung der allgemeinen Formel III

$$R'_5—Z \quad (III)$$

umsetzt, worin Z ein reaktives Halogenatom Cl, Br oder Jod oder ein reaktiver Sulfosäurerest bedeutet und $R'_5$ die weiter oben genannte Bedeutung für $R_5$ besitzt oder eine Alkoxycarbonylalkyl-Gruppe mit 3 bis 8 C-Atomen darstellt, und in der erhaltenen Verbindung der Formel IV

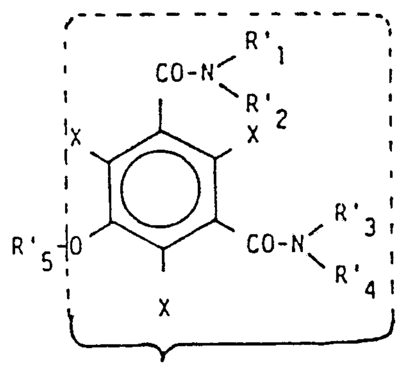

(IV)

worin die verschiedenen Symbole die weiter oben genannte Bedeutung besitzen, vorhandene 5-Alkoxycarbonylalkoxy-Gruppen durch Umsetzung mit einem primären und sekundären Amin in die entsprechende 5-Amino-carbonylalkoxy-Gruppe überführt und schliesslich Hydroxy-Funktionen maskierende Schutzgruppen durch Hydrolyse entfernt und die Verbindung der Formel I isoliert.

**Claims** (for the Contracting States : BE, CH, DE, FR, GB, IT, LI, LU, NL, SE)

1. 5-Alkoxy-2,4,6-triiodo- or tribromo-isophthalic diamides of the general formula (I)

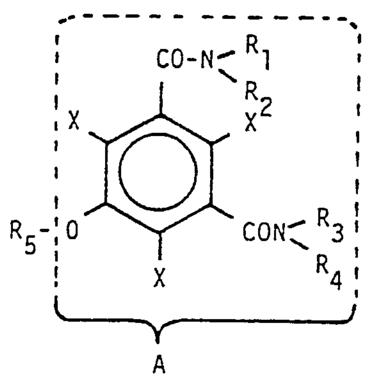

(I)

in which

X denotes iodine or bromine

$R_1$ and $R_3$ may be identical or different, namely denote hydrogen, lower alkyl or hydroxyalkyl,

$R_2$ and $R_4$ may be identical or different, namely denote hydroxyalkyl having 1 to 5 HO functions and 2 to 6 C atoms,

$R_5$ denotes hydroxyalkyl having 1 to 5 HO functions and 2 to 6 C atoms, denotes (poly) alkoxyalkyl having 1 to 10 oxa functions and 3 to 33 C atoms, denotes hydroxy(poly) alkyl having 1 to 10 oxa functions and 4 to 33 C atoms denotes aminocarbonylalkyl of the formula

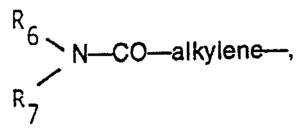

in which

$R_6$ denotes H, alkyl or hydroxyalkyl,

$R_7$ denotes hydroxyalkyl having 1 to 5 HO functions and 2 to 6 C atoms, and

alkylene denotes straight-chain or branched divalent alkylene having 1 to 5 C atoms, and

$R_5$ denotes alkyl having 10 to 20 C atoms, or A-alkylene'-, in which alkylene' denotes divalent alkylene having 2 to 12 C atoms and 0 to 5 oxa, thia, HO or amide functions, and A denotes a 3,5-bis-(aminocarbonyl)-2,4,6-trihalo-phenoxy radical of the formula I, which is located within the dashed line.

2. X-ray contrast agent containing a shadow-producing substance according to patent claim 1.

3. Process for the preparation of compounds of the general formula I, characterized in that a compound of the general formula II

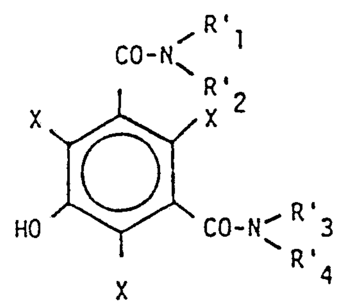

(II)

in which X, $R'_1$, $R'_2$, $R'_3$ and $R'_4$ have the abovementioned meaning for X, $R_1$, $R_2$, $R_3$ and $R_4$, but free hydroxyl groups present in the molecule may also be present in protected form, or an alkali metal salt or alkaline-earth metal salt thereof, is reacted, in a manner which is known per se, with a compound of the general formula III,

$$R'_5\text{—}Z \quad \text{(III)}$$

in which Z denotes a reactive halogen atom Cl, Br or iodine, or a reactive sulphonic acid radical, and $R'_5$ has the abovementioned meaning for $R_5$ or represents an alkoxycarbonyl alkyl group having 3 to 8 C atoms, and, in the resultant compound of the formula IV

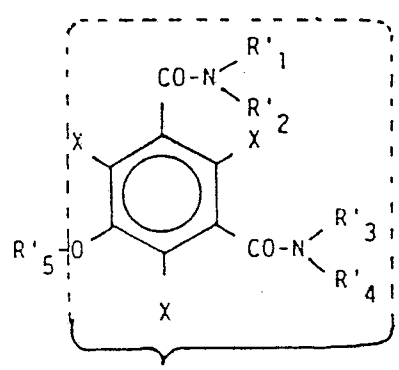

(IV)

A

in which the various symbols have the abovementioned meaning, 5-alkoxycarbonylalkoxy groups present are converted into the corresponding 5-aminocarbonylalkoxy group through reaction with a primary and secondary amine, and finally, protecting groups masking hydroxyl functions are removed by hydrolysis and the compound of the formula I is isolated.

**Claims** (for the Contracting State AT)

Process for the preparation of 5-alkoxy-2,4,6-triiodo- or tribromo-isophthalic diamides of the general formula (I).

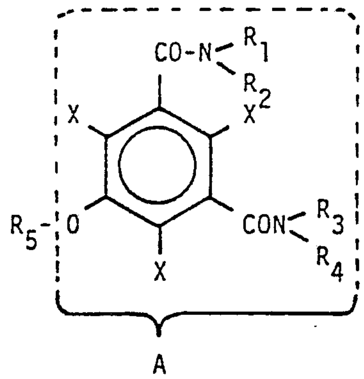

(I)

in which

X denotes iodine or bromine

$R_1$ and $R_3$ may be identical or different, namely denote hydrogen, lower alkyl or hydroxyalkyl,

$R_2$ and $R_4$ may be identical or different, namely denote hydroxyalkyl having 1 to 5 HO functions and 2 to 6 C atoms,

$R_5$ denotes hydroxyalkyl having 1 to 5 HO functions and 2 to 6 C atoms, denotes (poly) alkoxyalkyl having 1 to 10 oxa functions and 3 to 33 C atoms, denotes hydroxy(poly) alkyl having 1 to 10 oxa functions and 4 to 33 C atoms denotes aminocarbonylalkyl of the formula

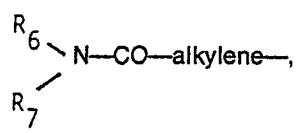

in which

$R_6$ denotes H, alkyl or hydroxyalkyl,

$R_7$ denotes hydroxyalkyl having 1 to 5 HO functions and 2 to 6 C atoms, and

alkylene denotes straight-chain or branched divalent alkylene having 1 to 5 C atoms, and

$R_5$ denotes alkyl having 10 to 20 C atoms, or A-alkylene'-, in which alkylene' denotes divalent alkylene having 2 to 12 C atoms and 0 to 5 oxa, thia, HO or amide functions, and A denotes a 3,5-bis-(aminocarbonyl)-2,4,6-trihalo-phenoxy radical of the formula I, which is located within the dashed line, characterized in that a compound of the general formula II

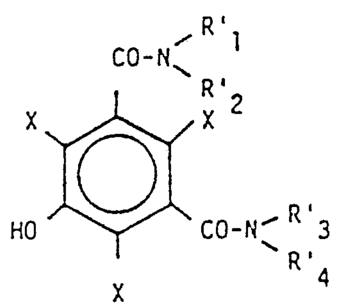

(II)

in which X, $R'_1$, $R'_2$, $R'_3$ and $R'_4$ have the abovementioned meaning for X, $R_1$, $R_2$, $R_3$ and $R_4$, but free hydroxyl groups present in the molecule may also be present in protected form, or an alkali metal salt or alkaline-earth metal salt thereof, is reacted, in a manner which is known per se, with a compound of the general formula III

$R'_5$—Z (III)

in which Z denotes a reactive halogen atom Cl, Br or iodine, or a reactive sulphonic acid radical, and $R'_5$ has the abovementioned meaning for $R_5$ or represents an alkoxycarbonyl alkyl group having 3 to 8 C atoms, and, in the resultant compound of the formula IV

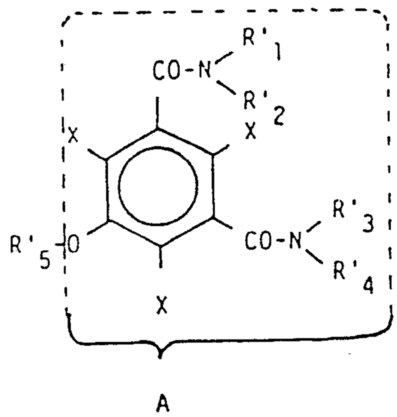

(IV)

in which the various symbols have the abovementioned meaning, 5-alkoxycarbonylalkoxy groups present are converted into the corresponding 5-aminocarbonylalkoxy group through reaction with a primary and secondary amine, and finally, protecting groups masking hydroxyl functins are removed by hydrolysis and the compound of the formula I is isolated.

**Revendications** (pour les Etats contractants : BE, CH, DE, FR, GB, IT, LI, LU, NL, SE)

1. Diamides d'acides 5-alcoxy-2,4,6-triiodo- et -tribromo-isophthaliques de formule générale I

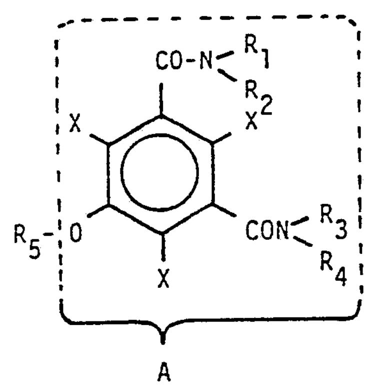

(I)

dans laquelle

X représente l'iode ou le brome,

$R_1$ et $R_3$ qui peuvent avoir des significations identiques ou différentes, représentent chacun l'hydrogène, un groupe alkyle inférieur ou hydroxyalkyle,

$R_2$ et $R_4$ qui peuvent avoir des significations identiques ou différentes, représentent chacun un groupe hydroxyalkyle contenant 1 à 5 fonctions OH et 2 à 6 atomes de carbone,

$R_5$ représente un groupe hydroxyalkyle contenant 1 à 5 fonctions OH et 2 à 6 atomes de carbone, un groupe (poly)alcoxyalkyle contenant 1 à 10 fonctions oxa et 3 à 33 atomes de carbone, un groupe hydroxy(poly)alkyle contenant 1 à 10 fonctions oxa et 4 à 33 atomes de carbone, un groupe aminocarbonylalkyle de formule

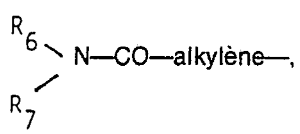

dans laquelle

$R_6$ représente H, un groupe alkyle ou hydroxyalkyle, et

$R_7$ représente un groupe hydroxyalkyle contenant 1 à 5 fonctions OH et 2 à 6 atomes de carbone, et

alkylène représente un groupe alkylène divalent à chaîne droite ou ramifiée en C 1-C 5,

$R_5$ représente un groupe alkyle en C 10-C 20, ou bien un groupe A-alkylène' — dans lequel « alkylène' » représente un groupe alkylène divalent en C 2-C 12 contenant 0 à 5 fonctions oxa, thia, HO ou amide et A est un groupe 3,5-bis-(aminocarbonyl)-2,4,6-trihalogéno-phénoxy répondant à la partie de la formule I qui se trouve à l'intérieur du trait interrompu.

2. Agent de contraste aux rayons X contenant une substance donnant les ombres selon la rev. 1.

3. Procédé de préparation des composés de formule générale I, caractérisé en ce que l'on fait réagir de manière connue en soi un composé de formule générale II

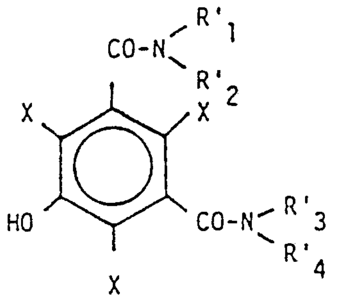

(II)

dans laquelle X, $R'_1$, $R'_2$, $R'_3$ et $R'_4$ ont les significations indiquées ci-dessus pour X, $R_1$, $R_2$, $R_3$ et $R_4$, mais les groupes hydroxy libres présents dans la molécule peuvent également être à l'état protégé, ou un sel alcalin ou alcalino-terreux d'un tel composé, avec un composé de formule générale III

$$R'_5—Z \qquad (III)$$

dans laquelle Z représente un atome d'halogène réactif Cl, Br ou I ou un radical acide sulfonique réactif et $R'_5$ a les autres significations indiquées ci-dessus pour $R_5$ ou représente un groupe alcoxycarbonylalkyle en C 3-C 8, ce qui donne un composé de formule IV

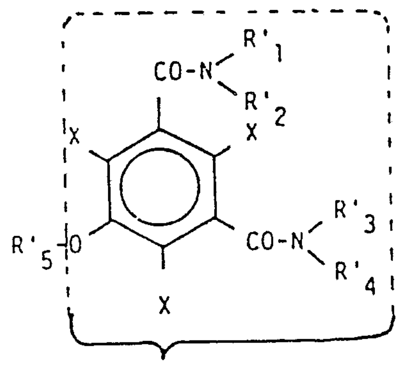

(IV)

dans laquelle les divers symboles ont les significations indiquées ci-dessus, dans lequel on convertit les groupes 5-alcoxycarbonylalcoxy présents, par réaction avec une amine primaire et secondaire, en le groupe 5-aminocarbonylalcoxy correspondant, et finalement, on élimine par hydrolyse les groupes protecteurs bloquant les fonctions hydroxy et on isole le composé de formule I.

**Revendication** (pour l'Etat contractant AT)

Procédé de préparation de diamides d'acides 5-alcoxy-2,4,6-triiodo- et -tribromo-isophtaliques de formule générale I

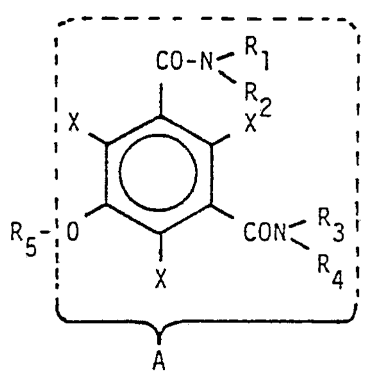

(I)

dans laquelle

X représente l'iode ou le brome,

$R_1$ et $R_3$ qui peuvent avoir des significations identiques ou différentes, représentent chacun l'hydrogène, un groupe alkyle inférieur ou hydroxyalkyle,

$R_2$ et $R_4$ qui peuvent avoir des significations identiques ou différentes, représentent chacun un groupe hydroxyalkyle contenant 1 à 5 fonctions OH et 2 à 6 atomes de carbone,

$R_5$ représente un groupe hydroxyalkyle contenant 1 à 5 fonctions OH et 2 à 6 atomes de carbone, un groupe (poly)alcoxyalkyle contenant 1 à 10 fonctions oxa et 3 à 33 atomes de carbone, un groupe hdyroxy(poly)alkyle contenant 1 à 10 fonctions oxa et 4 à 33 atomes de carbone, un groupe aminocarbonylalkyle de formule

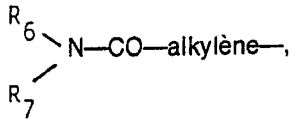

dans laquelle

$R_6$ représente H, un groupe alkyle ou hydroxyalkyle, et

$R_7$ représente un groupe hydroxyalkyle contenant 1 à 5 fonctions OH et 2 à 6 atomes de carbone, et alkylène représente un groupe alkylène divalent à chaîne droite ou ramifiée en C 1-C 5,

$R_5$ représente un groupe alkyle en C 10-C 20, ou bien un groupe A-alkylène' — dans lequel « alkylène' » représente un groupe alkylène divalent en C 2-C 12 contenant 0 à 5 fonctions oxa, thia, HO ou amide et A est un groupe 3,5-bis-(aminocarbonyl)-2,4,6-trihalogéno-phénoxy répondant à la partie de la formule I qui se trouve à l'intérieur du trait interrompu,

caractérisé en ce que l'on fait réagir de manière connue en soi un composé de formule générale II

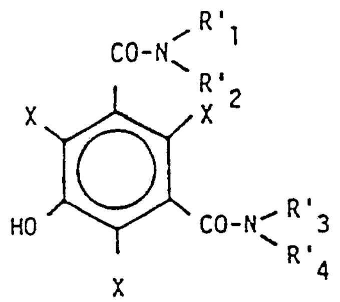

(II)

dans laquelle X, $R'_1$, $R'_2$, $R'_3$ et $R'_4$ ont les significations indiquées ci-dessus pour X, $R_1$, $R_2$, $R_3$ et $R_4$, mais les groupes hydroxy libres présents dans la molécule peuvent également être à l'état protégé, ou un sel alcalin ou alcalino-terreux d'un tel composé, avec un composé de formule générale III

$$R'_5—Z \qquad (III)$$

dans laquelle Z représente un atome d'halogène réactif Cl, Br ou I ou un radical acide sulfonique réactif et $R'_5$ a les autres significations indiquées ci-dessus pour $R_5$ ou représente un groupe alcoxycarbonylalkyle en C 3-C 8, ce qui donne un composé de formule IV

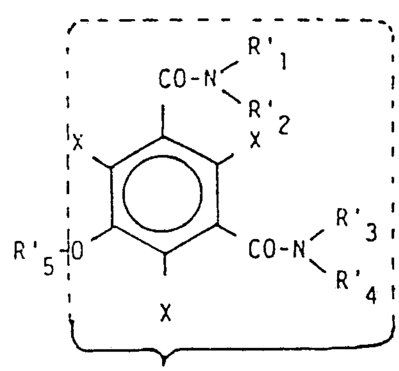

(IV)

A

dans laquelle les divers symboles ont les significations indiquées ci-dessus, dans lequel on convertit les groupes 5-alcoxycarbonylalcoxy présents, par réaction avec une amine primaire et secondaire, en le groupe 5-aminocarbonylalcoxy correspondant, et finalement, on élimine par hydrolyse les groupes protecteurs bloquant les fonctions hydroxy et on isole le composé de formule I.